# EUROPEAN PATENT SPECIFICATION

(11) **EP 3 125 970 B1**
(45) Date of publication and mention of the grant of the patent: **20.05.2020**
(21) Application number: 15773029.2
(22) Date of filing: 31.03.2015
(51) Int. Cl.: A61M 39/02, A61M 5/00, A61M 39/22

(54) **MEDICAL ACCESS PORT**
MEDIZINISCHER ZUGANGSPORT
ORIFICE D'ACCÈS MÉDICAL

(30) Priority: 31.03.2014 US 201414231392
(43) Date of publication of application: 08.02.2017
(73) Proprietor: Versago Vascular Access, Inc., West Bridgewater, Massachusetts 02379 (US)
(72) Inventor: TALLARIDA, Steven J., Mansfield, Massachusetts 02048 (US); RODGERS, Richard P., Hudson, Massachusetts 01749 (US); BUTZIGER, John M., East Greenwich, Rhode Island 02818 (US)
(74) Representative: Weisbrodt, Bernd
(86) International application number: PCT/US2015/023590
(87) International publication number: WO 2015/153611

(56) References cited:
- WO-A1-2007/051563
- US-A- 5 213 574
- US-A- 6 007 516
- US-A1- 2007 078 432
- US-A1- 2007 233 019
- US-A1- 2011 137 288
- US-A1- 2013 226 101
- US-A1- 2013 231 637
- US-B1- 7 056 316
- US-B2- 7 351 233

## Description

### FIELD

The present disclosure relates generally to medical access ports and, in particular, to subcutaneous vascular access ports that may include one or more needles that penetrates the skin, wherein the needle(s) may extend or retract from the access port, and a position of the needle(s) may change in the access port to change the penetration location of the skin.

### BACKGROUND

Hematology patients, oncology patients, hemodialysis patients and other patients may be subject to frequent infusion treatments delivering pharmaceuticals, blood, nutrients, contrasting agents and other compositions. Frequent "needle sticks" and the duration of infusion time may make receiving such treatments uncomfortable and creates scarring and added discomfort to the patient. Vascular access ports are medical devices that may be inserted beneath the skin (sub-cutaneous) and may reduce the discomfort associated with such treatments. A port may include an access point, such as a septum, into which a needle may be inserted. A port may also include a catheter, which may be inserted into a vein, such as a jugular vein, subclavian vein or superior vena cava. The septum may be formed of a self-healing silicone material that may be punctured multiple times with a relatively low loss in the integrity of the septum. However, a clinician needs to properly target the access port and a risk of infection may exist as a needle extending into the skin may drag bacteria from the skin into the port. US2011/137288 discloses a subcutaneous implantable access port having an access port body and at least one access needle extendable and retractable relative to the access port body connecting a fluid path with an internal catheter which allows access to the needle lumen for the passage of fluids.

### SUMMARY

Medical devices comprising implantable, subcutaneous, access ports are described for providing repeated therapy to a patient in need of such therapy. In certain embodiments, the access port, upon repeated use, may present an access needle at a new location/position of the access port each time the access port is utilized, so as to reduce and preferably minimize scarring injury to the tissue (skin) of the patient.

In certain embodiments, a medical device according to the present disclosure may include a subcutaneous access port having an access port body and at least one needle having a removable needle tip and a needle shaft defining a needle lumen; the at least one needle housed within the access port body, the at least one needle extendable and retractable relative to the access port body; and a needle shift mechanism operable such that the at least one needle is extendable from and retractable into the access port body at a plurality of positions of the access port body.

In certain embodiments, the needle shift mechanism may be operable such that the at least one needle is rotatable along an arcuate path about an axis of rotation. The arcuate path may be defined by a substantially constant radius from the axis of rotation, and may extend at least 90 degrees around the axis of rotation (e.g. at least 180 degrees around the axis of rotation, at least 270 degrees around the axis of rotation and 360 degrees around the axis of rotation)

In certain embodiments, the plurality of positions of the access port body may be arranged along the arcuate path, and the plurality of positions may be substantially equally spaced along the arcuate path.

In certain embodiments, the at least one needle may be rotatable along an arcuate path about an axis of rotation in only one direction.

In certain embodiments, the needle shift mechanism may comprise a ratcheting mechanism.

In certain embodiments, the access port body may comprise a cover; and the at least one needle may be extendable and retractable through the cover.

In certain embodiments, the access port body may comprise a septum; and the at least one needle may be extendable and retractable through the septum.

In certain embodiments, the access port body may comprise a plurality of needle openings; and the at least one needle may be extendable and retractable through each of the plurality of needle openings.

In certain embodiments, the plurality of needle openings may comprise a first needle opening and a second needle opening; and the needle shift mechanism may be operable to move the at least one needle from a first position in which the at least one needle is aligned with the first needle opening to extend and retract through the first needle opening to a second position in which the at least one needle is aligned with the second needle opening to extend and retract through the second needle opening.

In certain embodiments, the at least one needle having a removable tip and a needle shaft defining a needle lumen may further comprise a first needle having a removable first needle tip and a first needle shaft defining a first needle lumen, and a second needle having a removable second needle tip and a second needle shaft defining a second needle lumen; and the needle shift mechanism may be further operable such that the first needle is extendable from and retractable into the access port body at a plurality of first needle positions of the access port body, and the second needle is extendable from and retractable into the access port body at a plurality of second needle positions of the access port body.

In certain embodiments, the needle shift mechanism may be operable such that the first needle and the second needle are rotatable along an arcuate path about an axis of rotation.

In certain embodiments, the needle shift mechanism may be operable such that the first needle is rotatable along a first arcuate path about an axis of rotation, and the first needle is rotatable along a second arcuate path about the axis of rotation.

In certain embodiments, the needle shift mechanism may be operable with a button located on the access port.

In certain embodiments, the medical device may further comprise a needle elevator mechanism operable to extend the at least one needle from the access port body and retract the at least one needle into the access port body.

In certain embodiments, the access port body may comprise a cover having at least one opening; and the needle elevator mechanism may be operable to extend the at least one needle from the access port body through the at least one opening in the cover and retract the at least one needle into the access port body through the at least one opening in the cover.

In certain embodiments, the needle elevator mechanism may be operable to extend the at least one needle from the access port body and retract the at least one needle into the access port body by rotating the needle elevator mechanism about an axis of rotation.

In certain embodiments, the needle elevator mechanism may comprise an elevator member and a needle support member having the at least one needle supported thereon; and the elevator member may be threadably engaged with the needle support member and rotatable relative to the needle support member to extend the at least one needle from the access port body and retract the at least one needle into the access port body.

In certain embodiments, the elevator member may comprise a cylindrical member which is rotatable around the needle support member.

In certain embodiments, the elevator member may comprise a threaded rod which is rotatable in a threaded opening of the needle support member.

In certain embodiments, the needle elevator mechanism may comprise a ratcheting mechanism.

In certain embodiments, the needle elevator mechanism may comprise at least one magnet.

In certain embodiments, the at least one magnet may be magnetically engagable with an external actuator to extend the at least one needle from the access port body and retract the at least one needle into the access port body.

In certain embodiments, the at least one magnet is rotatable with the actuator to extend the at least one needle from the access port body and retract the at least one needle into the access port body.

In certain embodiments, the at least one magnet may be movable towards the actuator to extend the at least one needle from the access port body and movable away from the actuator to retract the at least one needle into the access port body.

In certain embodiments, the needle elevator mechanism may comprise a balloon arrangement.

In certain embodiments, the needle elevator mechanism may comprise a spring.

In certain embodiments, the needle elevator mechanism may be operable with a button located on the access port.

In certain embodiments, the medical device may further comprise at least one valve located within the access port to control a flow of fluid through the access port.

In certain embodiments, the at least one needle may comprise at least two needles; and the at least one valve may comprise a plurality of valves.

In certain embodiments, the medical device may further comprise a first channel bridging the at least two needles; and at least one of the plurality of valves arranged to open and close a flow of fluid across the first channel.

In certain embodiments,, the medical device may further comprise at least two catheters; a second channel bridging the at least two catheters; and at least one of the plurality of valves arranged to open and close a flow of fluid across the second channel.

In certain embodiments, the access port may include a body, a cover with a plurality of openings, at least one needle with a tip and a shaft, the shaft defining a lumen, a needle elevator mechanism to operate the position of the at least one needle in at least a retracted position in which the at least one needle is disposed in the body and the needle tip below the cover and an extended position in which the at least one needle is protruding through at least a first one of the openings, and a needle shift mechanism to move the at least one needle from a first position in which the needle is aligned with a first one of the openings, to a second position in which the needle is aligned with a second one of the openings.

In certain embodiments, the access port may include a holder having the at least one needle disposed thereon and helical threads and the elevator mechanism may include helical threads positioned to engage with the thread of the holder. In some embodiments, the elevator mechanism may include a cylinder having thread in an interior surface, the needles being disposed inside the cylinder. In some embodiments, the elevator mechanism may include a rod having thread on an exterior surface, the needles being disposed beside the rod. In some other embodiments, the elevator mechanism may include at least one magnet. In some embodiments, the at least one magnet is a permanent magnet, or an electromagnet.

In certain embodiments, the needle shift mechanism may include a rotatable surface and at least one guide rod, the guide rod guiding the motion of the needle when the needle is being extended or retracted, and positioning the needle from the first position to the second position. In some embodiments, the needle shift mechanism may further include a slot and pin combination to position the needle at one of the at least first and second position. In some embodiments, the needle shift mechanism may further include a ratchet mechanism to position the needle at one of the at least first and second position.

In certain embodiments, the access port may also include a button that engages the needle shift mechanism to position the needle at one of the at least first to a second position. In certain embodiments, the needle shift mechanism can move the at least one needle from about six to about twelve positions. In some embodiments, at least one of the positions is a maintenance position which provides access to a replaceable part of the access port.

In certain embodiments, the access port may also include at least a valve to close flow of fluid through the at least one needle. In some embodiments, the access port may also include at least two needles and two valves, and a first channel bridging the two needles, wherein the valves close or open flow of fluid between the first channel and the at least two needles. In some embodiments, the access port may also include at least two vascular catheter inlets and a second channel bridging the two catheter inlets, wherein the valves close or open flow of fluid between the first channel and the at least two needles and between the second channel and the at least two vascular catheter inlets.

In a certain embodiment, an access port according to the present disclosure and the invention(s) disclosed herein may include a body defining at least two recesses for defining at least a first position and a second position, a cover with a plurality of openings, at least one needle including a tip and a shaft, the shaft defining a lumen, a cylinder having threads on an interior surface with the needles disposed inside the cylinder, a holder having the at least one needle disposed thereon and threads disposed to engage the threads of the cylinder, at least one magnet engaged with the cylinder to operate the position of the at least one needle in at least a retracted position in which the at least one needle is disposed in the body and the needle tip below the cover and an extended position in which the at least one needle is protruding through at least a first one of the openings; and a base plate disposed in the body and having at least one flexible resilient prong engaging at least one of the at least two recesses of the body, the base plate engaging the at least one needle to move the at least one needle from the first position in which the needle is aligned with a first one of the openings, to the second position in which the needle is aligned with a second one of the openings. In some embodiment, the base plate may further include at least a guide rod for engaging the at least one needle from the first position to the second.

In another embodiment, an access port according to the present disclosure and the invention(s) disclosed herein may include a body defining at least two recesses for defining at least a first position and a second position, a cover comprising a plurality of openings, at least one needle with a tip and a shaft, the shaft defining a lumen, a rod disposed along an axis of rotation of the access port and having threads on an exterior surface, a holder having the at least one needle disposed thereon and threads disposed to engage the threads of the rod, at least one magnet engaged with the rod to operate the position of the at least one needle in at least a retracted position in which the at least one needle is disposed in the body and the needle tip below the cover and an extended position in which the at least one needle is protruding through at least a first one of the openings, and a base plate disposed in the body and having at least one flexible resilient prong engaging at least one of the at least two recesses of the body, the base plate engaging the at least one needle to move the at least one needle from the first position in which the needle is aligned with a first one of the openings, to the second position in which the needle is aligned with a second one of the openings. In some embodiments, the base plate may further include at least a guide rod for engaging the at least one needle from the first position to the second.

In some other embodiments, the access port may include a body, a cover including a plurality of openings at least one needle having a tip and a shaft, the shaft defining a lumen, a holder for carrying the at least one needle inside the port, a ratchet unit disposed along an axis of the access port and having a bottom portion having at least two teeth and processes and having a top portion defining at least two teeth and processes, the bottom teeth and processes capable of engaging the top teeth and processes defining at least a first position and a second position, a first button engaging the holder to operate the position of the at least one needle in at least a retracted position in which the at least one needle is disposed in the body and the needle tip below the cover and an extended position in which the at least one needle is protruding through at least a first one of the openings, and a second button engaging the ratchet unit to move the at least one needle from the first position in which the needle is aligned with a first one of the openings, to the second position in which the needle is aligned with a second one of the openings. In some embodiments, the top ratchet portion may further include at least two slots for engaging at least one pin, wherein when the pin is sliding along one of the slot, the at least one needle is raised or lowered, and when the pin is shifted from one of the slot to the other one, the at least one needle is shifted from the first position to the second position.

In yet other embodiments, the access port may include a body, a cover comprising a plurality of openings, at least two needles, each comprising a tip and a shaft, the shaft defining a lumen, a holder for carrying the needles inside the port, a ratchet unit disposed along an axis of the access port and having a bottom portion having at least two teeth and processes and having a top portion defining at least two teeth and processes, the bottom teeth and processes capable of engaging the top teeth and processes defining at least a first position and a second position, a magnet engaging the holder to position the needles from at least a retracted position in which the needles are disposed in the body and the needle tips below the cover to an extended position in which the needles are engaged through at least a first two of the openings, a coil spring to operate the holder to position the needles from at least an extended position in which the needles are engaged through at least two openings to a retracted position in which the needles are disposed in the body and the needle tips below the cover, at least two valves, a first channel bridging the two needles, wherein the valves close or open flow of fluid between the first channel and the at least two needles, at least two vascular catheter inlets, and a second channel bridging the two catheter inlets, wherein the valves close or open flow of fluid between the first channel and the at least two needles and between the second channel and the at least two vascular catheter inlets; and a button engaging the holder to move the at least one needle from the first position in which the needle is aligned with a first one of the openings, to the second position in which the needle is aligned with a second one of the openings.

In yet some embodiments, there is provided a system to access the vasculature of a patient. The system may include an access port and an actuator as described below. The access port may include a body defining at least two recesses for defining at least a first position and a second position, a cover comprising a plurality of openings, at least one needle comprising a tip and a shaft, the shaft defining a lumen, a cylinder having threads on an interior surface, the needles being disposed inside the cylinder, a holder having the at least one needle disposed thereon and threads disposed to engage the threads of the cylinder, at least one magnet engaged with the cylinder to operate the position of the at least one needle in at least a retracted position in which the at least one needle is disposed in the body and the needle tip below the cover and an extended position in which the at least one needle is protruding through at least a first one of the openings, and a base plate disposed in the body and having at least one flexible resilient prong engaging at least one of the at least two recesses of the body, the base plate engaging the at least one needle to move the at least one needle from the first position in which the needle is aligned with a first one of the openings, to the second position in which the needle is aligned with a second one of the openings. The actuator may include a magnet, for positioning over the skin of a patient above the implanted access port. In some embodiments, the actuator magnet is an electromagnet.

In yet other embodiments, there is provided a system to access the vasculature of a patient. The system may include an access port and an actuator as described below. The access port may include a body defining at least two recesses for defining at least a first position and a second position, a cover comprising a plurality of openings, at least one needle comprising a tip and a shaft, the shaft defining a lumen, a rod disposed along an axis of rotation of the access port and having threads on an exterior surface, a holder having the at least one needle disposed thereon and threads disposed to engage the threads of the rod, at least one magnet engaged with the rod to operate the position of the at least one needle in at least a retracted position in which the at least one needle is disposed in the body and the needle tip below the cover and an extended position in which the at least one needle is protruding through at least a first one of the openings, and a base plate disposed in the body and having at least one flexible resilient prong engaging at least one of the at least two recesses of the body, the base plate engaging the at least one needle to move the at least one needle from the first position in which the needle is aligned with a first one of the openings, to the second position in which the needle is aligned with a second one of the openings. The actuator may include a magnet, for positioning over the skin of a patient above the implanted access port. In some embodiments, the actuator magnet is an electromagnet.

### BRIEF DESCRIPTION OF THE DRAWINGS

The above-mentioned and other features of this disclosure, and the manner of attaining them, may become more apparent and better understood by reference to the following detailed description of embodiments described herein taken in conjunction with the accompanying drawings, wherein:
**FIG. 1a** illustrates a cross-sectional view of an example of a vascular access port contemplated herein, with the needles in the partially extended position;
**FIG. 1b** illustrates a cross-sectional view of the vascular access port of **FIG. 1a** paired with an actuator, with the needles in the retracted position;
**FIG. 1c** illustrates a cross-sectional view of the internal parts of the vascular access port of **FIG. 1a**, without the cover and the magnets;
**FIG. 1d** illustrates a cross-sectional top view of the vascular access port of **FIG. 1a**, at the base plate level showing detail of the ratcheting mechanism;
**FIG. 2a** illustrates a cross-sectional view of an example of a vascular access port contemplated herein paired with an actuator, showing an example of a ratcheting mechanism;
**FIGS. 2b** and **2d** illustrate cross-sectional views of the ratcheting mechanism of **FIG. 2a****,** in various configurations;
**FIG. 3a** illustrates a perspective view of an alternate example of a vascular access port contemplated herein, with the needles in the retracted position;
**FIG. 3b** illustrates a side view of an example of the ratcheting mechanism as shown in **FIG. 3a****;**
**FIGS. 3c to 3e** illustrate cross-sectional views of the ratcheting mechanism in various configurations according to the vascular access port as shown in **FIG. 3a****;**
**FIG. 4a** illustrates a partial cross-sectional view of the needle arm of the vascular access port as shown in **FIG. 3a****,** with the needles in the retracted position;
**FIG. 4b** illustrates a partial cross-sectional view of the needle arm of the vascular access port as shown in **FIG. 3a****,** with the needles in the extended position;
**FIG. 5a** illustrates a perspective view of a vascular access port as shown in **FIG. 3a****;**
**FIG. 5b** illustrates a side view of the operation of the needles of a vascular access port as shown in **FIG. 5a****,** in a blood flow mode;
**FIG. 5c** illustrates a perspective view of a vascular access port as shown in **FIG. 5a****;**
**FIG. 5d** illustrates a side view of the operation of the needles of a vascular access port as shown in **FIG. 5a****,** in a needle-cleaning mode;
**FIG. 6** illustrates a cross-sectional view of an example of a vascular access port contemplated herein paired with an actuator, with the needles in the retracted position;
**FIG. 7a** illustrates a perspective view of an example of a vascular access port contemplated herein, with the needles in the retracted position;
**FIG. 7b** illustrates a perspective view of an example of a vascular access port contemplated herein paired with an actuator, with the needles in the extended position;
**FIG. 7c** illustrates a cross-sectional view of an example of the vascular access port shown in **FIG. 7a****,** with the needles in the retracted position;
**FIG. 7d** illustrates a cross-sectional view of an example of the vascular access port shown in **FIG. 7b****,** with the needles in the extended position;
**FIG. 8a** illustrates a side view of an example of a ratcheting mechanism according to the vascular access port shown in **FIGS. 7c****-d;**
**FIGS. 8b** to **8d** illustrate side views of the ratcheting mechanism various configurations as shown in **FIG. 8a****;**
**FIG. 9a** illustrates a cross-section view of a vascular access port as shown in **FIGS. 7a** to **7d****,** in a blood flow mode;
**FIG. 9b** illustrates a side view of the operation of the needles of a vascular access port as shown in **FIG. 9a****,** in a blood flow mode;
**FIG. 9c** illustrates a perspective view of a vascular access port as shown in **FIG. 7a** to **7b****,** in a needle-cleaning mode;
**FIG. 9d** illustrates a side view of the operation of the needles of a vascular access port as shown in **FIGS. 9c****,** in a needle-cleaning mode;
**FIG. 10a** illustrates a cross-sectional view of an example of a vascular access port contemplated herein, with the needles in the retracted position;
**FIG. 10b** illustrates a cross-sectional view of the vascular access port of **FIG. 10a****,** with the needles in the extended position;
**FIG. 11** illustrates a perspective view of an example of a vascular access port contemplated herein, with the needles in the extended position;
**FIG. 12** illustrates a perspective view of the ratcheting mechanism as shown in **FIG. 11****,** with the needles in the retracted position;
**FIG. 13** illustrates a perspective view of a portion of the ratcheting mechanism as shown in **FIG. 12****,** with the needle in the retracted position;
**FIGS. 14a** and **14b** illustrate top views of the configurations of a torsion spring used in the ratcheting mechanism as shown in **FIG. 12****;**
**FIG. 15** illustrates a perspective view of the ratcheting mechanism as shown in **FIG. 12****,** with the needles in the retracted position;
**FIG. 16** illustrates a perspective view of the ratcheting mechanism as shown in **FIG. 12****,** with the needles in the retracted position;
**FIGS. 17a** and **17b** illustrate top views of the various configurations of a torsion spring used in the ratcheting mechanism as shown in **FIG. 12****;**
**FIGS. 18a** to **18c** illustrate side views of the various configurations of the ratcheting mechanism as shown in **FIG. 12****;**
**FIG. 19** illustrates a cross-sectional view of an example of a vascular access port contemplated herein, with the needles in extended position, and flow path open; and
**FIG. 20** illustrates a cross-sectional view of the vascular access port of **FIG. 19****,** with the needles in extended position, and flow path closed.

### DETAILED DESCRIPTION

It is to be understood that this disclosure is not limited in its application to the details of construction and the arrangement of components set forth in the following description or illustrated in the drawings. The embodiments herein are capable of other embodiments and of being practiced or of being carried out in various ways within the scope of the claims.

Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not restricted to physical or mechanical connections or couplings.

Spatial references such as "above," "below," "top," "bottom," "horizontal," "vertical," "right," "left," and the like are meant to be understood in relation to the orientation of the device and parts thereof as illustrated in the figure being described, and are interchangeable upon spatial reorientation of the device.

Embodiments herein may refer to motion of moving parts as clockwise or counter-clockwise. Such embodiments should not be regarded as limiting of the invention as mirror-image embodiments can be adapted to perform the same operation or function in a reverse sense of motion, counterclockwise or clockwise as appropriate.

The present disclosure relates to sub-cutaneous vascular access ports that may be connected to a blood vessel, body cavity or organ of a patient via one or more internal (indwelling) catheters. The access ports include at least one extendable/retractable needle. For certain medical applications, such as hemodialysis, two needles may be required for the exit of blood from the patient and return of the clean blood to the patient. The embodiments of the access ports described herein focusing on ports having two needles are equally applicable to ports having one needle, or more than two needles. The needles may puncture through the skin of a patient, providing access to the port. A catheter or other device may be affixed to the needles protruding from the port and through the skin of the patient to deliver a composition to the patient. The needles may also puncture a vial stopper to deliver a composition stored in the vial.

In general, the access ports may provide one or more extendable/retractable needles operated by a needle elevator mechanism. In addition, the access ports may also provide multiple positions for the one or more needles to extend/retract, so that the one or more needles will extend/retract at a new position for each use. The one or more needles may be shifted, particularly by being rotated, from a first position to the next position by a needle shift mechanism inside the port. The needle elevator mechanism may be operated with an external actuator, or with an internal actuator within the port. Similarly, the shift mechanism may be operated with an external actuator, or with an internal actuator within the port.

Examples of suitable external actuators may include permanent magnets or electromagnets. Examples of suitable internal actuators may include buttons, levers, switches and the like.

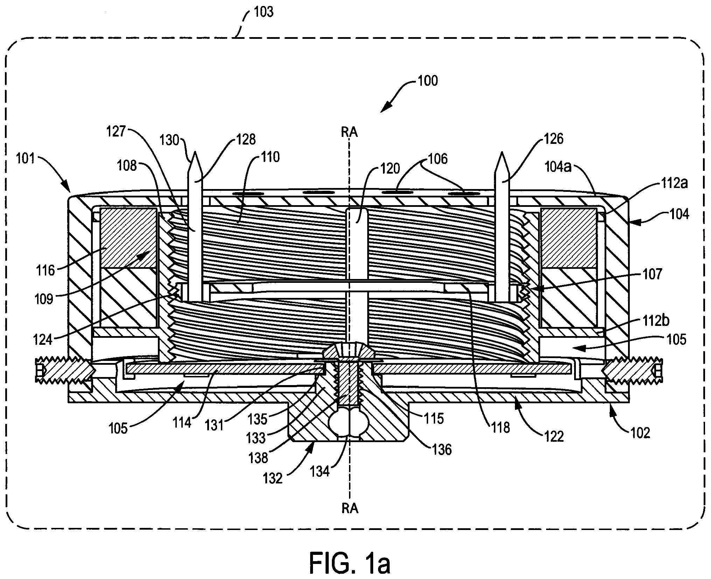

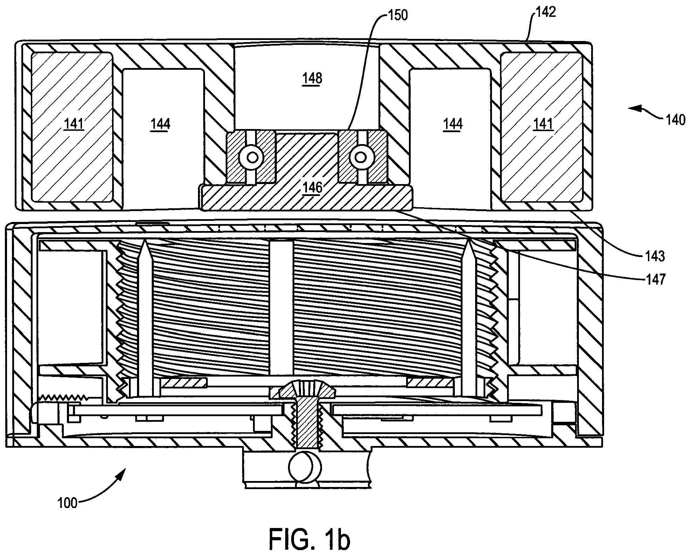

**FIGS. 1a** and **1b** illustrate an example of an access port **100** which may include at least one needle **126,** and more particularly two needles **126.** In **FIG. 1a**, the two needles **126** are shown in an at least partially extended (exposed) position, and in **FIG. 1b**, the two needles are shown in a fully retracted (concealed) position. In such regards, it may be understood that the needles **126** travel axially (parallel) along a center/rotational axis **RA** of the access port **100** as described herein.

The needles **126** may include a hollow shaft **128** for connecting a fluid path with an internal catheter accessing a blood vessel (not shown), and a closed, pointed, removable tip **130** which allows access to a lumen **127** of the needles **126** for the passage of fluids (e.g. medication, blood). The needles **126** may be made of medical grade steel, or of a ferromagnetic material, or may include a ferromagnetic material at the tip **130.**

**FIG. 1a** illustrates the access port **100** may further include a body **101** comprising a base **102,** generally having a shape of a circular plate, supporting an overlying cylindrical cover **104,** which cooperate to form an inner cavity **105.** Base **102** includes a hub **132** that serves as the connection area to which catheters or other devices used may be attached. Details of such connections are well known and not illustrated here.

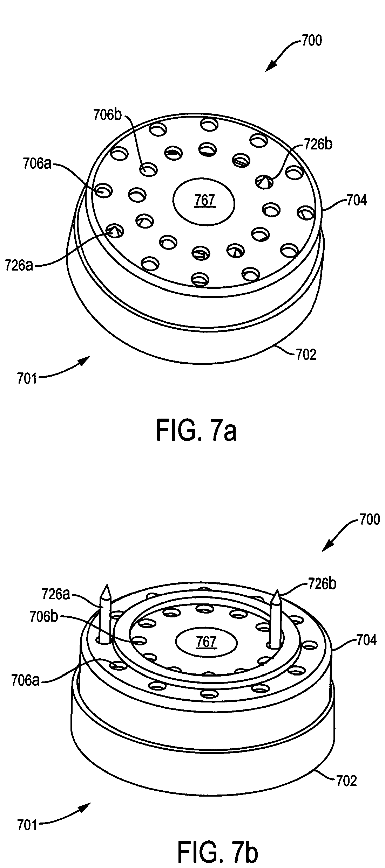

The cover **104** may include on the top surface/wall **104a** a series of predefined openings **106** to allow the passage of needles **126** through the top **wall 104a** of the cover **104** to retract into and extend out of the body **101** of the access port **100.** As shown, for example, in **FIG. 7a****,** the openings **106** may be arranged in a circular (arcuate) ring pattern and, in certain embodiments, in the form of two concentric (arcuate) rings as an outer ring and an inner ring.

In order to prevent access of body fluids and/or ingrowth of tissue into the openings **106** in the access port **100,** the access port **100** may be contained in a housing **103** (shown in phantom), which may be made of a "self-healing" material, such as silicone. When extended, the needles **126** may pierce through the self-healing material. Thereafter, when the needles **126** are retracted, the openings **106** created in the self-healing material may sufficiently close (i.e. self-close) so that the access port **100** remains impervious to body fluids and ingrowth of body tissue. Alternatively, the cover **104,** and more particularly the top wall **104a** of the cover **104,** may be made of a self-healing material, and the needles **126** may pierce through the top wall **104a** of the cover **104,** which may sufficiently close (i.e. self-close) after the needles **126** are retracted to inhibit ingress of to body fluids and ingrowth of body tissue.

The cavity **105** of the access port **100** may contain a needle elevator mechanism **107,** which may comprise a cylindrical (elevator) member **109,** which may rests and rotate on a circular floor **114.** As explained herein, the floor **114** of the needle elevator mechanism **107** may also operate as part of a needle shift mechanism **122.** As shown, the base **102** supports floor **114** and provides the structural foundation for the needle elevator mechanism **107** and the needle shift mechanism **122,** as well as forms part of the needle shift mechanism **122.**

To secure the floor **114** and the base **102** to one another, the floor **114** may include a center circular opening **115** having a diameter to receive a spindle **131** formed by a threaded, shouldered, screw boss **133** of the hub **132** of the base **102.** Once on the spindle **131,** floor **114** may rest on the shoulder **135** of the boss **133** and be secured to the boss **133** by a threaded screw fastener **138** (and a washer/seal **134**) which couples with the threads **136** of boss **133,** and retains the floor **114** perpendicular to the screw boss **133.** As explained in greater detail below, the floor **114,** and more particularly the needle elevator mechanism **107** and the needle shift mechanism **122,** may rotate about an axis of rotation **RA** of the access port **100,** here provided by the spindle **131,** where the elevator mechanism moves the needles **126** axially along the axis of rotation **RA** and the needle shift mechanism **122** moves the needles circumferentially (radially) around the axis of rotation **RA.**

Cylindrical (elevator) member **109** of the needle elevator mechanism **107** comprises a cylinder **108** which may include internal threads **110,** as well as outer peripheral flanges **112,** provided by upper flange **112a** and lower flange **112b.** The flanges **112a, 112b** may be used to hold at least one permanent magnet **116** in a fixed position there between.

The needle elevator mechanism **107** may further comprise a needle support member **118.** The access port needles **126** may be secured to (fastened) and supported within the interior of the cylinder **108** by the needle support member **118,** which generally has a shape of an annular plate. One or more elongated guide post members **120** maintain a pre-selected circumferential (radial) position of the needles **126** in the access port **100** during an operation of the needle elevator mechanism **107** that elevates and retracts the needles **126,** in and out of the body **101** of the access port **100.** The needle support member **118** may have external threads **124** on the periphery that engage the internal threads **110** of the cylinder **108.**

**FIG. 1b** further illustrates the access port **100** paired with a separate actuator **140.** The actuator **140** may include at least one magnet **141** (e.g. permanent magnet or an electromagnet) in body **142,** which may be used as a handle, as well as a circular chamber **144** that receives the needles **126** in the extended configuration. The actuator **140** may include bearings **150** disposed in a central bore **148** between the body **142** and a center rotational support **146,** which ease rotation of the body **142** relative to the rotational support **146.** As shown, the front (skin contact) face **147** of the rotational support **146** is offset (stepped) and projects outwardly relative to the front surface **143** of the body **142** to provide a gap/spacing to keep the main body **142** from overly contacting the surface of the skin or sterile covering for the skin of the patient and easing rotation of the body **142** of the actuator **140.**

In operation, the access port **100** may be positioned under the skin of a host, particularly a patient, such as a patient particularly in need of repeated vascular access. Medical personnel (e.g. physician, clinician) may then position the actuator **140** onto the skin above (overlying) the implanted access port **100,** at which time the magnets **141** placed within body **142** may operate the needle elevator mechanism **107** inside the access port **100.**

When the magnets **141** of the actuator **140** are electromagnets, and the magnets **116** of the access port **100** are permanent magnets, the actuator magnets **141** magnetically engage with the magnets **116** within the port **100** and induce rotation of the cylindrical (elevator) member **109.** More particularly, when an electric current of a first polarity is provided to electromagnet **141,** electromagnet **141** may emit an electro-magnetic field arranged with a first polarity which attracts the permanent magnet **116** of the access port **100.** Thereafter, rotation of the body **142** of the actuator **140** about rotational support **146** will correspondingly result in rotation of the cylindrical (elevator) member **109** of the access port **100** about the rotational axis **RA.**

The rotation of the cylindrical (elevator) member **109** causes the sliding rotation of the external threads **124** of the needle support member **118** along the internal threads **110** of the cylinder **108,** moving the needles **126** upward or downward along the axis of rotation **RA** depending on the direction of the rotation of the cylindrical (elevator) member **109.** For example, counter-clockwise rotation of the body **142** of the actuator **140** and cylindrical (elevator) member **109** of the access port **100** may elevate the needle support member **118** and needles **126,** while clockwise rotation of the body **142** of the actuator **140** and cylindrical (elevator) member **109** of the access port **100** may retract the needle support member **118** and needles **126.**

**FIG. 1a** illustrates the access port **100** with the needles **126** in the partially extended position with the tips **130** of the needles **126** slightly protruding from the cover **104** of the access port **100.** **FIG. 1b** illustrates the access port **100** with the needles **126** in the retracted position before actuation of the access port **100.** Among other benefits, the mechanical engagement provided by threads **110** and **124** allows the needles **126** to be arranged in a fully extended (axial) position, a fully retracted (axial) position, or be partially extended/retracted at any (axial) position there between.

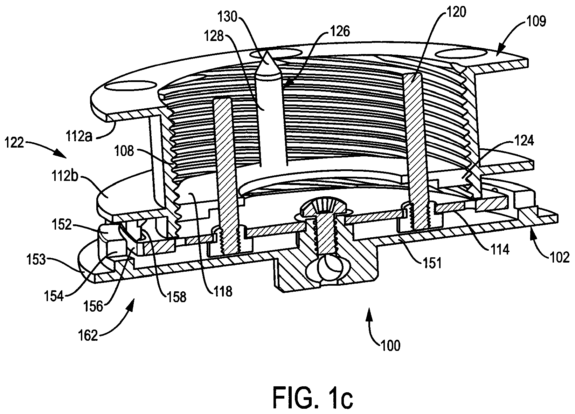

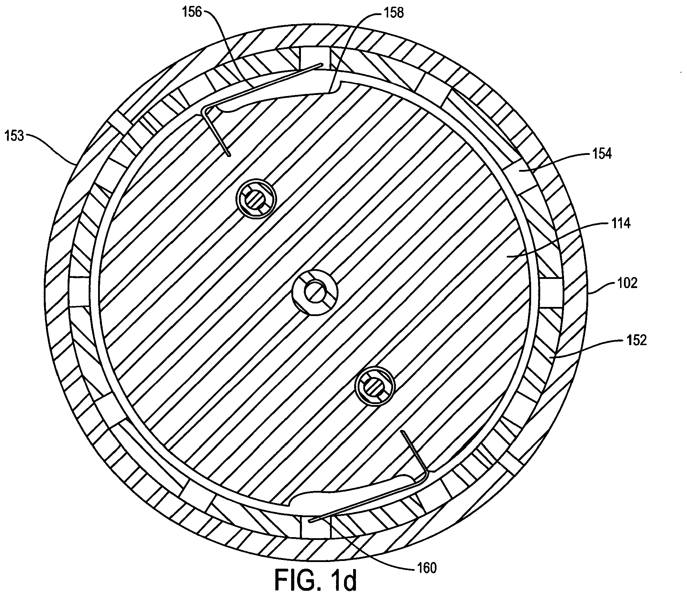

The access port **100** may also include a needle shift mechanism **122** that, upon full retraction of the needles **126** into the vascular port **100,** shifts/rotates a circumferential (radial) position of the needles **126** inside the cylinder **108** of the cylindrical (elevator) member **109** around the axis of rotation **RA** provided by the spindle **131** such that, upon subsequent use and reactivation of the access port **100,** the needles **126** will protrude from the port at a new, different location through the cover **104.** One example of such needle shift mechanism **122** is described below and illustrated in **FIGS. 1c** and **1d****.**

Referring to **FIGS. 1c** and **1d****,** the access port **100** may include a needle shift mechanism **122,** and more particularly a ratcheting mechanism **162,** that may move the position of the needles **126** within the access port **100** at the end of each use, so that, on the next use of the access port **100,** the needles **126** will pierce the skin at a new, different location and minimize the patient discomfort due to excessive scarring which may occur when the needles **126** exit the skin repeatedly at the same location.

In **FIG. 1c**, the base **102** may include a circular base portion **151** (which extends substantially transverse to the axis of rotation **RA** of the needle elevator mechanism **107** and the needle shift mechanism **122**), as well as a raised circular rim **152** which extends substantially transverse to the circular base portion **151** (i.e. substantially parallel to the axis of rotation **RA**). As shown, circular rim **152** is located proximally adjacent the peripheral edge **153** of the circular base portion **151** of the base **102** and surrounds the floor **114.**

The circular rim **152** may include a plurality of slots **154** which, as explained in greater detail herein, predetermine the various circumferential (radial) positions that the needles **126** may occupy in the access port **100.** In certain embodiments, there may be from two to twenty slots **154,** and more particularly six to twelve slots **154,** that may be uniformly spaced apart at regularly spaced intervals (i.e. the same) around the circular rim **152.** The number of slots **154** may depend on the intended repeated use of the access port **100,** with more slots **154** corresponding to greater use thereof. In the displayed embodiment, the circular rim **152** includes twelve slots **154** which afford six positions for two needles **126,** or twelve positions for one needle **126,** to pierce the skin of a patient at a new, different location.

The ratcheting mechanism **162** further includes at least one flexible and resilient (deformable) engagement member **156,** such as a linear spring (which may be deformed towards recess **158**), connected to or integral (i.e. one monolithic piece) with the floor **114,** which may engage with any one of the slots **154.** As shown, the engagement members **156** are arranged to engage a slot **154** at an engagement angle such that, the floor **114** may be rotated in one direction but not the other direction. More particularly, when the floor **114** is rotated in one direction, but not in the opposite direction, the terminal end (extremity) **160** of the engagement members **156** abuts (contacts) a vertical sidewall of the slot **154** in which it resides.

Due to the angle of engagement and the configuration of the engagement members **156,** the engagement members **156** prevent the floor **114** from rotating in one direction (e.g. the clockwise direction) when the needles **126** are being raised (due to the terminal end **160** contacting the vertical sidewall of the slot **154**). Conversely, the engagement members **156** allow the floor **114** to rotate in the opposite direction (e.g. the counter-clockwise direction) by deforming inward against the circular rim **152** and out of the slot **154** once the needles **126** have been fully retracted and the needle support member **118** frictionally engages with the floor **114.** At this point, the floor **114** rotates counter-clockwise from a first circumferential (radial) position to the next available circumferential (radial) position afforded by the slots **154.** Alternately, the direction of the threads **110, 124** of cylinder **108** and needle support member **118,** respectively, and the design of the ratcheting mechanism shown in **FIG. 1d** could be reversed to make the ratcheting mechanism rotate in the opposite direction.

As set forth above, the needle shift mechanism **122,** and more particularly the ratcheting mechanism **162,** may operate by friction of the needle support member **118** onto the floor **114,** or by the engagement of a pin/notch combination (not shown) positioned between the needle support member **118** and the floor **114** so as to engage one another once the needle support member **118** has reached is lowermost position in the access port **100** against the floor **114.**

With the foregoing needle shift mechanism **122,** and more particularly ratcheting mechanism **162** such as one detailed in **FIG. 1d****,** rotation of the floor **114** may be understood to stop at one of the defined positions for subsequent extension and retraction. The ratcheting mechanism **162** may provide an auditory signal (e.g. a "click") when it reaches one of the defined circumferential (radial) positions. The design allows medical personnel to further rotate the needles **126** to other further circumferential (radial) positions if it is so desired to avoid a sensitive location for the patient.

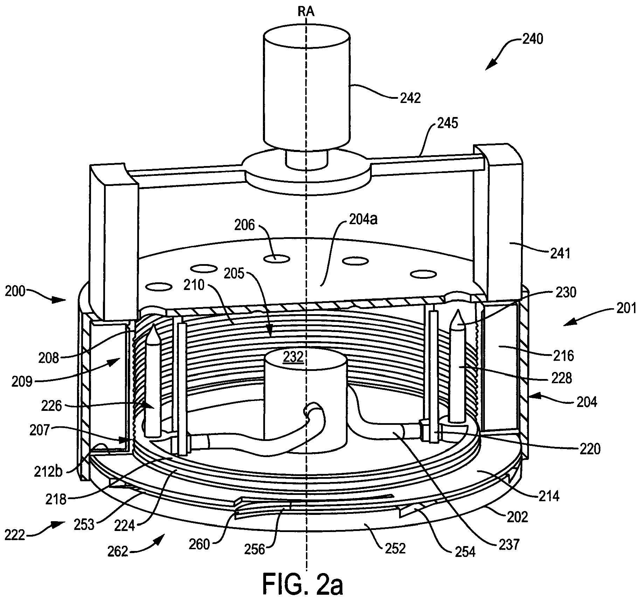

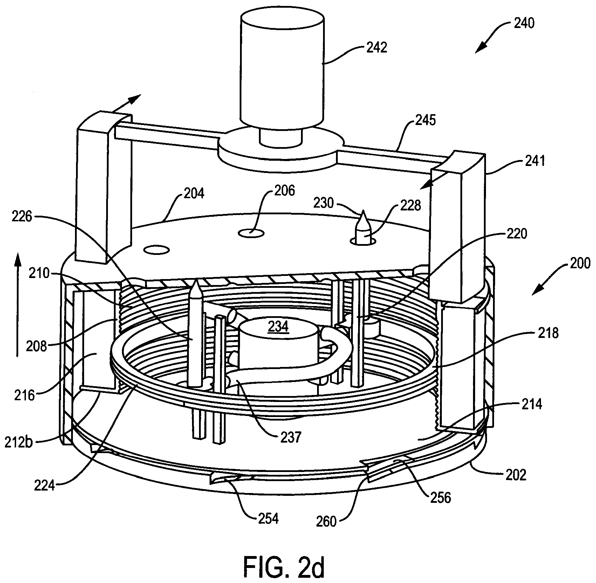

**FIGS. 2a** to **2d** illustrate another example of an access port **200** with an alternate needle shift mechanism **222,** and more particularly a ratcheting mechanism **262.** As with the first embodiment, **FIG. 2a** illustrates the access port **200** may include a base **202** supporting an overlying cover **204,** which cooperate to form an inner cavity **205.**

Also as with the first embodiment, the cover **204** may include on the top surface/wall **204a** a series of predefined openings **206** to allow the passage of needles **226** through the top wall **204a** of cover **204** to retract into and extend out of the body **201** of the access port **200.** The needles **226** may include a hollow shaft **228** for connecting a fluid path with an internal catheter accessing a blood vessel (not shown), and a closed, pointed, removable tip **230** which allows access to a lumen **227** of the needles **226** for the passage of fluids (e.g. medication, blood). The needles **226** may be made of medical grade steel, or of a ferromagnetic material, or may include a ferromagnetic material at the tip **230.**

As with the prior embodiment, in order to prevent access of body fluids and/or ingrowth of tissue into the openings **206** in the access port **200,** the access port **200** may be contained in a housing **203** (not shown, similar to housing **103**), which may be made of a "self-healing" material, such as silicone. When extended, the needles **226** may pierce through the self-healing material. Thereafter, when the needles **226** are retracted, the openings **206** created in the self-healing material may sufficiently close (i.e. self-close) so that the access port **200** remains impervious to body fluids and ingrowth of body tissue. Alternatively, the cover **204,** and more particularly the top wall **204a** of the cover **204,** may be made of a self-healing material, and the needles **226** may pierce through the top wall **204a** of the cover **204,** which may sufficiently close (i.e. self-close) after the needles **226** are retracted to inhibit ingress of to body fluids and ingrowth of body tissue.

The cavity **205** of the access port **200** may contain a needle elevator mechanism **207,** which may comprise a cylindrical (elevator) member **209,** which rests and rotates on circular floor **214.** As explained herein, the floor **214** of the needle elevator mechanism **207** may also operate as part of the needle shift mechanism **222.** As shown, the base **202** supports floor **214** and provides the structural foundation for the needle elevator mechanism **207** and the needle shift mechanism **222,** as well as forms part of the needle shift mechanism **222.** As explained in greater detail below, the floor **214,** and more particularly the needle elevator mechanism **207** and the needle shift mechanism **222,** may rotate about an axis of rotation **RA** of the access port **200.**

Cylindrical (elevator) member **209** of the needle elevator mechanism **207** comprises a hollow cylinder **208** which includes internal threads **210,** as well as outer peripheral flanges **212** provided by upper flange **212a** and lower flange **212b.** The flange **212a, 212b** may be used to support and hold at least one permanent magnet **216** in a fixed position there between.

The needle elevator mechanism **207** further comprises a needle support member **218.** The access port needles **226** are secured to (fastened) and supported within the interior of the cylinder **208** by the needle support member **218.** One or more elongated guide post members **220** maintain a pre-selected circumferential (radial) position of the needles **226** in the access port **200** during an operation of the needle elevator mechanism **207** that elevates and retracts the needles **226,** in and out of the body **201** of the access port **200.** The needle support member **218** may have external threads **224** on the periphery that engage the internal threads **210** of the cylinder **208.** The needles **226** may each include a shaft **228** connected to a flexible linking tube **237** connected a central (post) hub **232.** The central hub **232** may include channels that connect to catheters that access the blood vessel (not shown).

In operation, the access port **200** may be positioned under the skin of a patient (such as a patient particularly in need of repeated vascular access) and operated with an actuator **240** overlying the skin. The actuator **240** may comprise a center hub **242,** which may provide a cylindrical body/handle which is located on and extends along the axis of rotation **RA.** The actuator **240** further comprises two laterally extending arms **245** which extend transverse to the axis of rotation **RA** on opposing sides of the hub **242.** Each arm **245** may support and hold at least one permanent magnet **241** fixed to the arm **245.**

Medical personnel (e.g. physician, clinician) may position the actuator **240** onto the skin above (overlying) the access port **200,** at which time the magnets **241** may operate the needle elevator mechanism **207** inside the access port **200.**

As set forth with the first embodiment, the actuator magnets **241** magnetically engage with the magnets **216** within the access port **200** and induce rotation of the cylindrical (elevator) member **209.** The rotation of the cylindrical (elevator) member **209** causes the sliding rotation of the external threads **224** of the needle support member **218** along the internal threads **210** of the cylinder **208,** moving the needles **226** upward or downward along the axis of rotation **RA** depending on the direction of the rotation of the cylindrical (elevator) member **209.**

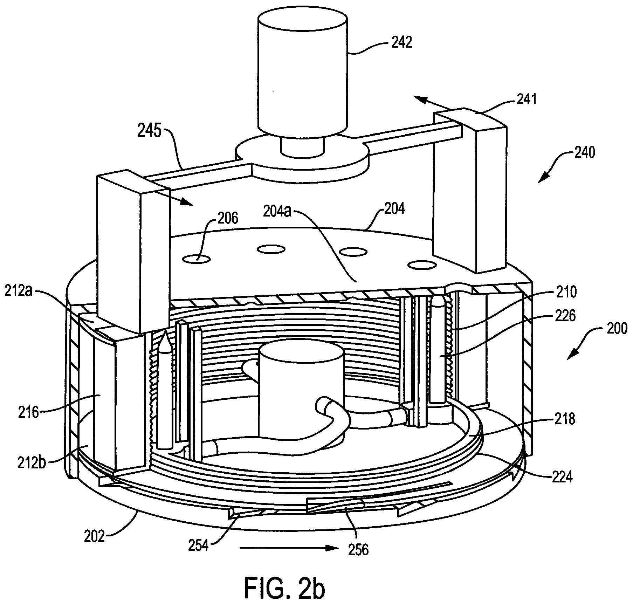

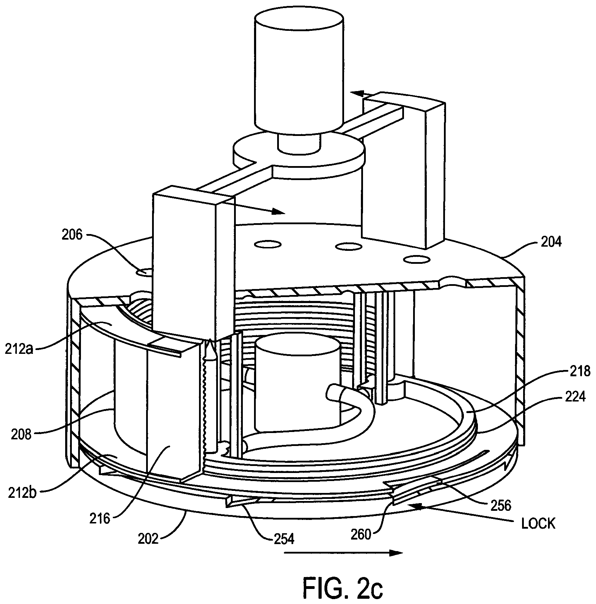

**FIGS. 2a****-d** illustrate the sequence of motions generated by the needle shift mechanism **222** inside the access port **200** that moves (rotates) the needles from a first circumferential (radial) position (**FIG. 2a**) to a second circumferential (radial) position (**FIG. 2c**). **FIG. 2a** illustrates the access port **200** with the needles **226** in the retracted position at a first circumferential (radial) position of the access port **200** after use. **FIG. 2b** illustrates the access port **200** with the needles **226** in transit and being rotated to a second circumferential (radial) position. **FIG. 2c** illustrates the access port **200** with the needles **226** at the second circumferential (radial) position.

As shown in **FIGS. 2a-2d****,** the needle shift mechanism **222** is operable such that the needles **226** are rotatable along an arcuate path about an axis of rotation **RA.** In the illustrated embodiment, the arcuate path is defined by a substantially constant radius (while access port **200** may be designed to rely upon a constant radius, the radius may vary slightly due to operational tolerances of the needle shirt mechanism) from the axis of rotation **RA.** The arcuate path my extent at least 90 degrees around the axis of rotation **RA,** and particularly at least 180 degrees around the axis of rotation **RA,** and more particularly at least 270 degrees around the axis of rotation **RA,** and even more particularly 360 degrees around the axis of rotation **RA.** The plurality of predetermined circumferential (radial) positions of the access port body **201** are arranged along the arcuate path, and more particularly the plurality of positions are substantially equally spaced along the arcuate path.

Moreover, the needle shift mechanism **222** is operable to move the needles **226** from a first position in which the needles **226** are aligned with the first needle opening to extend and retract through the first needle opening to a second position in which the needles **225** are aligned with the second needle opening to extend and retract through the second needle opening.

In **FIG. 2a****,** the base **202** may include at the peripheral edge **253** including plurality of slots **254** which predetermine the various circumferential (radial) positions that the needles **226** may occupy in the access port **200**. In certain embodiments, there may be from two to twenty slots **254,** and more particularly six to twelve slots **254,** that may be uniformly (equally) spaced apart at regularly spaced intervals (i.e. the same) around the peripheral edge **252.** The number of slots **254** may depend on the intended repeated use of the access port **200,** with more slots **254** corresponding to greater use thereof. In the displayed embodiment, the peripheral edge **253** includes twelve slots **254** which afford six circumferential (radial) positions for two needles **226,** or twelve positions for one needle **126,** to pierce the skin of a patient at a new, different location.

The ratcheting mechanism further includes at least one flexible and resilient (deformable) engagement member **256,** which is shown as a bent portion integral (i.e. one monolithic piece) with the floor **214,** which may engage with any one of the slots **254.** As shown, the engagement members **256** are arranged to engage a slot **254** at an engagement angle such that the terminal end (extremity) **260** of the engagement members **256** abuts (contacts) a vertical sidewall of the slot **254** in which it resides.

Due to the angle of engagement and the configuration of the engagement members **256,** the engagement members **256** prevent the floor **214** from rotating in one direction (e.g. the clockwise direction) when the needles **226** are being raised (due to the terminal end **260** contacting the vertical sidewall of the slot **254**). Conversely, the engagement members **256** allow the floor **214** to rotate in the opposite direction (e.g. the counter-clockwise direction) by deforming upwardly once the needles **226** have been fully retracted and the needle support member **218** frictionally engages with the floor **214.** At this point, the floor **214** rotates counter-clockwise from a first circumferential (radial) position to the next available circumferential (radial) position afforded by the slots **254.** Alternatively, the design of the ratcheting mechanism could be reversed to rotate in the opposite direction.

As set forth above, the needle shift mechanism **222,** and more particularly the ratcheting mechanism **262** may operate by friction of the needle support member **218** onto the floor **214,** or by the engagement of a pin/notch combination (not shown) positioned between the needle support member **218** and the floor **214** so as to engage one another once the needle support member **224** has reached is lower most position in the access port **200** against the floor **214.**

With the foregoing needle shift mechanism **222,** and more particularly ratcheting mechanism **262** such as one detailed in **FIG. 2a****,** rotation of the floor **214** may be understood to stop at one of the defined positions for subsequent extension and retraction. The ratcheting mechanism **262** may provide an auditory signal (e.g. a "click") when it reaches one of the defined circumferential (radial) positions. The design allows the medical personnel (e.g. physician, clinician) to further rotate the needles **226** to other further circumferential (radial) positions if it is so desired, such as to avoid a sensitive location for the patient.

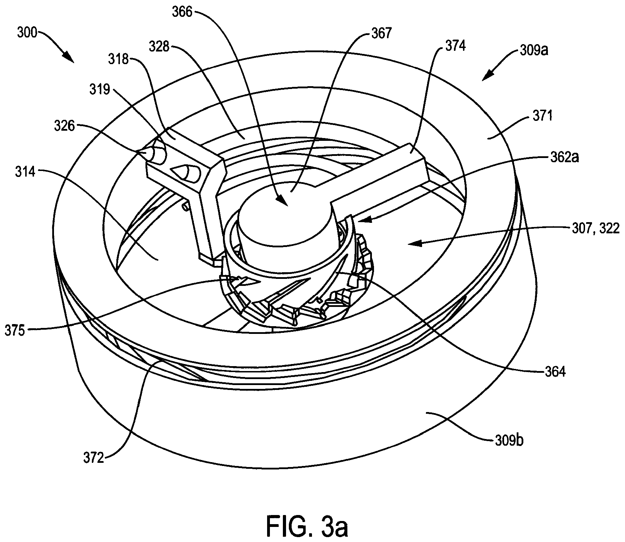

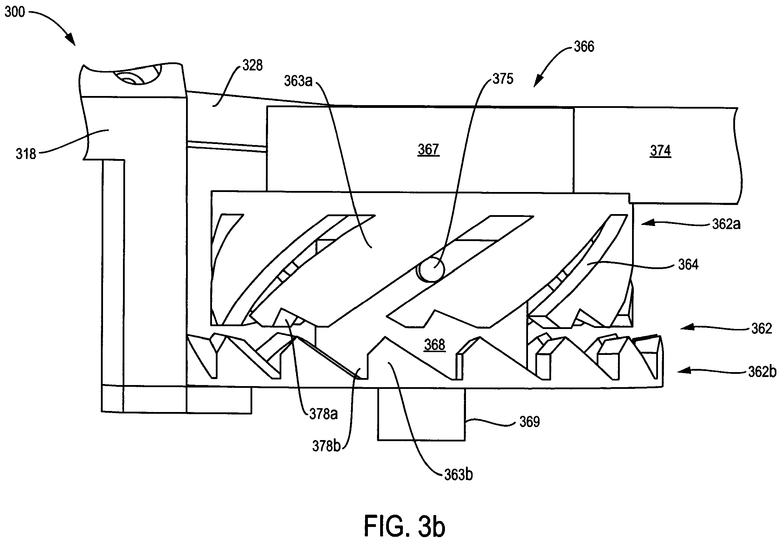

**FIGS. 3a** and **3b** illustrate another example of an access port **300.** Similar to the previous embodiments, the needle elevator mechanism **307** and the needle shift mechanism **322** may both rotate around an axis of rotation **RA** to extend/retract the needles **326** axially, as well as to change a circumferential (radial) position of the needles **326.** In this embodiment, the cover **304** has been removed to expose the needle elevator mechanism **307** and needle shift mechanism **322.** More particularly, the needle elevator mechanism **307** and the needle shift mechanism **322** may both operate via a circular ratcheting mechanism **362** disposed in the center of the access port **300,** which may comprise an upper (top) annular cylindrical ratchet portion **362a** disposed above a lower (bottom) annular cylindrical ratchet portion **362b** which engage and disengage from each other to extend/retract the needles **326** and change the circumferential (radial) position of the needles **326.**

As best shown by **FIG. 3b****,** inside the access port **300,** the needles **326** may be supported by a needle (arm) support member **318** that is connected (fixed) to the lower ratchet portion **362b** of a ratcheting mechanism **362.** More particularly, the needle support member **318** is disposed circumferentially alongside ratchet teeth **363b** of lower ratchet portion **362b.** The upper ratchet portion **362a** includes ratchet teeth **363a** which are separated by elongated openings (which may also be referred to as cam slits or slots) **364,** which are oblique to the axis of rotation **RA.**

Access port **300** further includes an internal actuator **366,** as opposed to the separate actuator used for the prior embodiments. Internal actuator **366** comprises a cylindrical push button **367** disposed over a hub **368** which moves (strokes) circumferentially on central spindle (shaft) **369** located within the confines of circular ratcheting mechanism **362.**

Button **367** may be depressed by pushing down on cover **304** (which overlies button **367**), at which time hub **368** (which is disposed beneath button **367**) may move downward, with hub **368** moving downward along spindle **369.** Furthermore, cover **304** may apply a compression force to the circular flange **371** of inner cylindrical member **309a** and move inner cylindrical member **309a** downward into outer cylindrical member **309b.** The depression is biased by a coil spring **372** disposed beneath cylindrical flange **371** between inner cylindrical member **309a** and outer cylindrical member **309b,** which returns the cover **304**/push button **367** to its pre-depressed position after the depression force is removed. As will become more apparent with further reading of the disclosure, the coil spring **372** serves to separate upper (top) ratchet portion **362a** and lower (bottom) annular ratchet portion **362b** until the button **367** is depressed.

The upper ratchet portion **362a** further includes a laterally projecting arm **374** that holds the needle shafts **328.** The arm **374** fits into a slot of the upper edge of the upper ratchet portion **362a,** so as to move it rotationally along with the needles **326.** The arm **374** contains fluid flow channels (not shown) connected and in fluid communication with the lumens **327** of needle shafts **328** at one end, and to the (vascular) catheters (not shown) at the other end. More particularly, the fluid flow channels direct flow for each needle **326** to a catheter supplying each needle **326** at the bottom of the access port **300.**

As shown, hub **368** may include at least one cam pin **375** which protrudes outwardly (transverse) to the axis of rotation **RA,** and which may be located in elongated cam slot opening **364.** When the button **367** is depressed, particularly by the medical personnel, such may operates the needle elevator mechanism **307** and the needle shift mechanism **322,** particularly via ratcheting mechanism **362.** More particularly, as button **367** begins to travel downward, hub **368** and cam pin **375** may simultaneously travel downward, particularly without rotating. Due to the cam slots **364** being arranged oblique to the axis of rotation **RA,** as cam pin **375** travels downward, it forces upper ratchet portion **362a** to rotate counter-clockwise as the cam pin **375** travels downward in cam slot **364.** Due to counter-clockwise rotation of upper ratchet portion **362,** the adjoining laterally projecting arm **374** also rotates counter-clockwise, at which time needle shafts **328** of needles **326,** extend through needle extension and retraction through-holes **319** formed in needle support member **318.** As shown, the needle extension and retraction through-holes **319** is also oblique to the axis of rotation **RA.** As such, when needle shafts **328** of needles **326,** which may comprise flexible semi-rigid tubing, are forced through through-holes **319** by the counter-clockwise rotation of upper ratchet portion **362,** the needles extend and rise through the through-holes **319** to operate the needle elevator mechanism **307.**

As button **367** continues to travel downward, upper (top) annular cylindrical ratchet portion **362a** and lower (bottom) annular cylindrical ratchet portion **362b** are brought into engagement with the upper ratchet teeth **363a** and lower ratchet teeth **363b** contacting to operate the needle shift mechanism **322.** More particularly, the notches **378a** of the upper ratchet teeth **363a** of upper ratchet portion **362a** engage with the notches **378b** of the lower ratchet teeth **363b** of lower ratchet portion **362b** forcing the lower ratchet portion **362b** to rotate clockwise one notch at a time for each depression of the button **367,** rotating with it the needle support member **318** clockwise and thus positioning the needles **326** at a new circumferential (radial) position to operate the needle shift mechanism **322.**

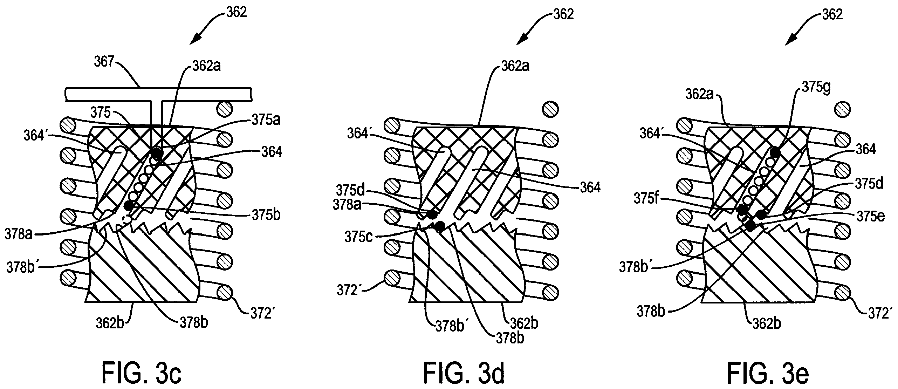

**FIGS. 3c** to **3e** illustrate various stages of the operation of the ratcheting mechanism **362** of access port **300** shown in **FIG. 3a****.** In the first stage, shown in **FIG. 3c****,** the upper ratchet portion **362a** and lower ratchet portion **362b** of the ratcheting mechanism **362** are disposed inside a coil spring **372'** similar to coil spring **372.** For simplicity, button **367** is shown directly connected to cam pin **375** engaged in cam slot **364** in the upper ratchet portion **362a.** When the button **367** is depressed the first time, cam pin **375** travels down cam slot **364** from position **375a** to **375b** until it reaches the bottom of notch **378b** of the bottom lower ratchet portion **362b** where it stops downward progress at position **375c.** During this time, as set forth above, the upper ratchet portion **362a** rotates counter-clockwise to operate the needle elevator mechanism **307** and, when teeth **363a, 363b** engage, the lower ratchet portion **362b** rotates clockwise to operate the needle shift mechanism **322.**

As such, upon release of the button **367,** the cam pin **375** now shifts (transfers) to notch **378a** of the upper ratchet portion **362a** at position **375d** where it lodges and maintains the button **367** in a depressed configuration as shown in **FIG. 3d****,** and needles **326** in an extended (raised) position. Referring to **FIG. 3e****,** when the button is depressed a second time, the cam pin **375** shifts (transfers) to position **375e** engaging notch **378b'** of the lower ratchet portion **362b** disposed clockwise of notch **378a** of upper ratchet portion **362a.** Upon release of the button **367** again, the cam pin **375** shifts (transfers) into adjacent cam slot **364'** at position **375f** of the upper ratchet portion **362a,** disposed clockwise to cam slot **364** to settle at and travels to the top of cam slot **364'** at position **375g,** during which time the upper ratchet portion rotates clockwise and needles **326** return to their retracted (lowered) position. As the can pin **375** travels from position **375a** to **375g,** the ratcheting mechanism **362** rotates the needles clockwise into a new position inside the access port **300.**

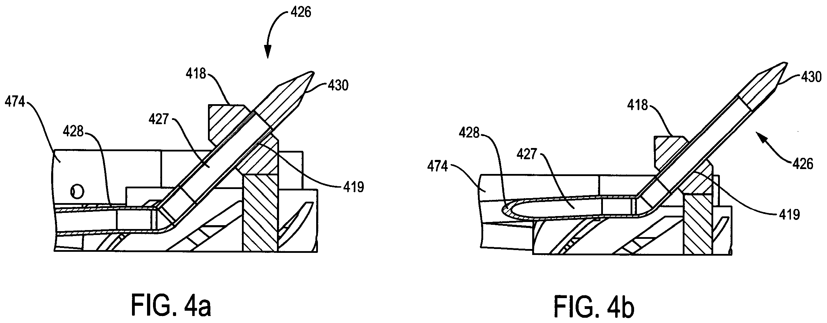

**FIGS. 4a** and **4b** illustrate the retracted and extended position of the needles **426** in relation to the needle support member **418** with a portion of the needle shaft **428** in cross-section to show the lumen **427** of the needle **426.** As the needle arm **474** rotates counter-clockwise, the proximal portion of the needle shaft **428** is pushed upward through through-holes **419** formed in needle support member **418,** lifting the tips **430** of the needles **426.**

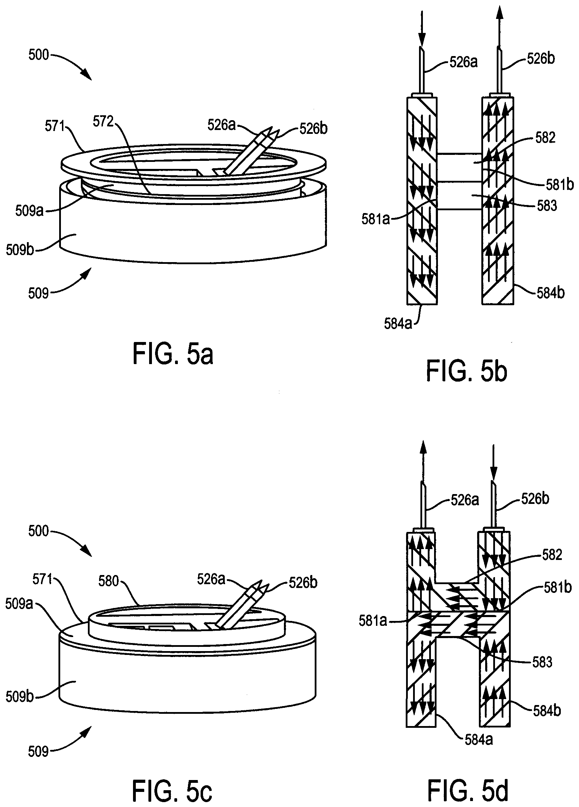

**FIG. 5a** illustrates a perspective view of the interior of an access port **500** similar to the port shown in **FIG. 3a****.** As set forth with the prior embodiment, a cylindrical member **509** may comprise an inner cylindrical member **509a** and an outer cylindrical member **509b** having a coil spring **572** located therebetween.

As shown in **FIG. 5a****,** coil spring **572** is decompressed and the circular flange **571** of inner cylindrical member **509a** is flush with the top rim of a smaller annular (cylindrical) frame **580.** The inner cylindrical member **509a** is propped in an upward position with coil spring **572.** In this configuration, the needles **526a** and **526b** operate in blood flow mode, as shown in **FIG. 5b****,** where valve **581a** of needle **526a** is open and in a vertical position preventing access to top bridge **582** and bottom bridge **583.** The same is observed for needle **526b,** where valve **581b** of needle **526b** is open and in a vertical position preventing access to top bridge **582** and bottom bridge **583.** The flow of blood can thus enter needle **526a** and exit needle **526b** as when the access port **500** is used for hemodialysis of a patient with kidney failure.

**FIG. 5c** illustrates a configuration in which coil spring **572** is compressed and the circular flange **571** of the inner cylindrical member **509a** is depressed and flush to the rim of the outer cylindrical member **509b.** In this configuration, the needles **526a** and **526b** operate in cleaning mode, as shown in **FIG. 5d****,** where valve **581a** of needle **526a** and valve **581b** of needle **526b** are closed and prevent the blood flow in needles **526a** and **526b** creating a lower loop for the blood flow from catheter **584b** to catheter **584a** in bottom bridge **583,** and allow access to top bridge **582** from the needles **526b** and **526a** for a cleansing solution, which may be used to prevent the formation of blood clots in the needles **526a** and **526b.**

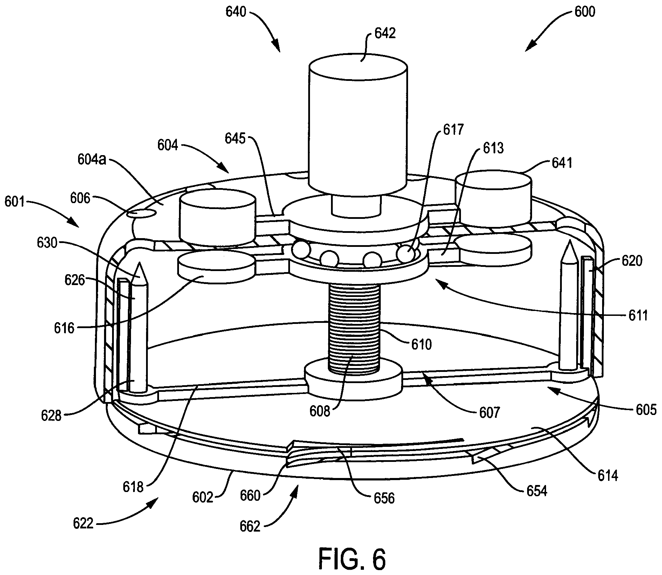

**FIG. 6** illustrates another example of an access port **600** also with a needle shift mechanism **622,** and more particularly a ratcheting mechanism **662.** As with prior embodiments, the access port **600** may include a base **602** supporting an overlying cover **604,** which cooperate to form an inner cavity **605.**

As with prior embodiments, the cover **604** may include on the top surface/wall **604a** a series of predefined openings **606** to allow the passage of needles **626** through the top wall **604a** of cover **604** to retract into and extend out of the body **601** of the access port **600.** The needles **626** may include a hollow shaft **628** for connecting a fluid path with an internal catheter accessing a blood vessel (not shown), and a closed, pointed, removable tip **630** which allows access to a lumen of the needles **626** for the passage of fluids (e.g. medication, blood). The needles **626** may be made of medical grade steel, or of a ferromagnetic material, or may include a ferromagnetic material at the tip **630.**

As with prior embodiments, in order to prevent access of body fluids and/or ingrowth of tissue into the openings **606** in the access port **600,** the access port **600** may be contained in a housing **603** (not shown, similar to housing **103**), which may be made of a "self-healing" material, such as silicone. When extended, the needles **626** may pierce through the self-healing material. Thereafter, when the needles **626** are retracted, the openings **606** created in the self-healing material may sufficiently close (i.e. self-close) so that the access port **600** remains impervious to body fluids and ingrowth of body tissue. Alternatively, the cover **604,** and more particularly the top wall **604a** of the cover **604,** may be made of a self-healing material, and the needles **626** may pierce through the top wall **604a** of the cover **604.**

The cavity **605** of the access port **600** contains a needle elevator mechanism **607,** which rests and rotates on circular floor **614.** As explained herein, the floor **614** of the needle elevator mechanism **607** may also operate as part of the needle shift mechanism **622.** As shown, the base **602** supports floor **614** and provides the structural foundation for the needle elevator mechanism **607** and the needle shift mechanism **622,** as well as forms part of the needle shift mechanism **622.** As explained in greater detail below, the floor **614,** and more particularly the needle elevator mechanism **607** and the needle shift mechanism **622,** may rotate about an axis of rotation **RA** of the access port **600.**

Needle elevator mechanism **607** comprises a center (elevator) hub/member **611** which provides a center rotational support. Center (elevator) hub/member **611** includes a threaded cylinder (rod) **608,** which includes external threads **610** and rotates about an axis of rotation **RA** of the access port **600.** Needle elevator mechanism **607** further comprises two laterally extending arms **613** which extend transverse to the axis of rotation **RA** on opposing sides of the center (elevator) hub/member **611.** Each arm **613** may support and hold at least one permanent magnet **616** fixed to the arm **612.** The center (elevator) hub/member **611** may include a ball bearing system **617** to facilitate the rotation of the magnets **616** in the access port **600** while extending (raising) and retracting (lowering) the needles **626.**

The needle elevator mechanism **607** further comprises a needle support member **618.** The access port needles **626** are secured to (fastened) and supported within the cavity **605** by the needle support member **618.** One or more elongated guide member **620** maintain a pre-selected circumferential (radial) position of the needles **626** in the access port **600** during an operation of the needle elevator mechanism **607** that elevates and retracts the needles **626,** in and out of the body **601** of the access port **600.** The needle support member **618** may have internal threads **624** at the center that engage the external threads **610** of the central threaded rod **608.** The needles **626** may each include a shaft **628** to connect, through a fluid path, with a catheter accessing the blood vessel (not shown).

In operation, the access port **600** may be positioned under the skin of a patient (such as a patient particularly in need of repeated vascular access) and operated with an actuator **640** overlying the skin. The actuator **640** may comprise a center hub **642,** which may provide a cylindrical body/handle which is located on and extends along the axis of rotation **RA.** The actuator **640** further comprises two laterally extending arms **645** which extend transverse to the axis of rotation **RA** on opposing sides of the hub **642.** Each arm **645** may support and hold at least one permanent magnet **641** fixed to the arm **245.**

Medical personnel (e.g. physician, clinician) may position the actuator **640** onto the skin above the access port **600,** at which time the magnets **641** may operate the needle elevator mechanism **607** inside the access port **600.**

The actuator magnets **641** magnetically engage with the magnets **616** within the access port **600** and induce rotation of the central rod **608.** The rotation of the central rod **608** causes the sliding rotation of the internal threads **624** of the needle support member **618** along the external treads **610** of the central rod **608,** and with the help of the elongated guide members **620,** moving the needles **626** upward or downward along the access of rotation **RA** depending on the direction of the rotation of the central rod **608.**

The access port **600** may also include a needle shift mechanism **622** that, upon full retraction of the needles **626** into the access port **600,** shifts the circumferential (radial) position of the needles **626** inside the access port **600** such that upon a subsequent activation of the access port **600,** the needles **626** will protrude from the vascular access port **600** at a new circumferential (radial) position.

As shown before in **FIG. 6****,** such needle shift mechanism **622** may particularly comprise a ratcheting mechanism **662** that moves the position of the needles **626** within the access port **600** at the end of each use, so that on the next use of the access port **600,** the needles **626** will pierce the skin at a new location. The base **602** may include at the peripheral edge **653** a defined plurality of slots **654** which predetermine the various circumferential (radial) positions that the needles **626** may occupy in the access port **600.** In certain embodiments, there may be from two to twenty slots **654,** and more particularly six to twelve, slots **654,** that may be uniformly spaced apart at regularly spaced intervals (i.e. the same) around the peripheral edge **652.** The number of slots **654** may depend on the intended repeated use of the access port **600,** with more slots **654** corresponding to greater use thereof. In the displayed embodiment, the peripheral edge **653** includes twelve slots **654** which afford six circumferential (radial) positions for two needles **626,** or twelve positions for one needle **626,** to pierce the skin of a patient at a new, different location.

The ratcheting mechanism **662** further includes at least one flexible and resilient (deformable) engagement member **656,** which is shown as a bent portion integral (i.e. one monolithic piece) with the floor **614,** which may engage with any one of the slots **654.** As shown, the engagement members **656** are arranged to engage a slot **654** at an engagement angle such that the terminal end (extremity) **660** of the engagement members **656** abut (contacts) a vertical sidewall of the slot **654** in which it resides.

Due to the angle of engagement and the configuration of the engagement members **656,** the engagement members **656** prevent the floor **614** from rotating in one direction (e.g. the clockwise direction) when the needles **626** are being raised (due to the terminal end **660** contacting the vertical sidewall of the slot **654**). Conversely, the engagement members **656** allow the floor **614** to rotate in the opposite direction (e.g. the counter-clockwise direction) by deforming upwardly once the needles **626** have been fully retracted and the needle support member **618** frictionally engages with the floor **614.** At this point, the floor **614** rotates counter-clockwise from a first circumferential (radial) position to the next available circumferential (radial) position afforded by the slots **654.** Alternatively, the design of the ratcheting mechanism **662** could be reversed to rotate in the opposite direction.

The ratcheting mechanism **662** may operate by friction of the needle support member **618** onto the floor **614,** or by the engagement of a pin/notch combination (not shown) positioned between the needle support member **618** and the floor **614** so as to engage one another once the needle support member **624** has reached is lowermost position in the access port **600.** Due to a ratcheting mechanism **662** such as one detailed in **FIG. 6****,** rotation of the floor **614** will tend to stop at one of the pre-defined positions for later needle extraction.

**FIGS. 7a** and **7b** illustrate another example of an access port **700** with a needle elevator mechanism **707** and needle shift mechanism **722.** The access port **700** may include a body **701** comprising a cupped cylindrical base **702** supporting an overlying cylindrical cover **704.** The cover **704** may include on the top surface/wall **704a** a series of predefined openings **706a, 706b** to allow the passage of needles **726a, 726b** respectively, through the top wall **704a** of the cover **704** to retract into and extend out of the body **701** of the access port **700.**

In order to prevent access of body fluids and/or ingrowth of tissue into the openings **706a, 706b** in the access port **700,** the access port **700** may also be contained in a housing **703** (not shown, similar to housing **103**), which may be made of a "self-healing" material, such as silicone. When extended, the needles **726a, 726b** may pierce through the self-healing material. Thereafter, when the needles **726a, 726b** are retracted, the openings **706a, 706b** created in the self-healing material may sufficiently close (i.e. self-close) so that the access port **700** remains impervious to body fluids and ingrowth of body tissue. Alternatively, the cover **704,** and more particularly the top wall **704a** of the cover **704,** may be made of a self-healing material, and the needles **726a, 726b** may pierce through the top wall **704a** of the cover **704.**

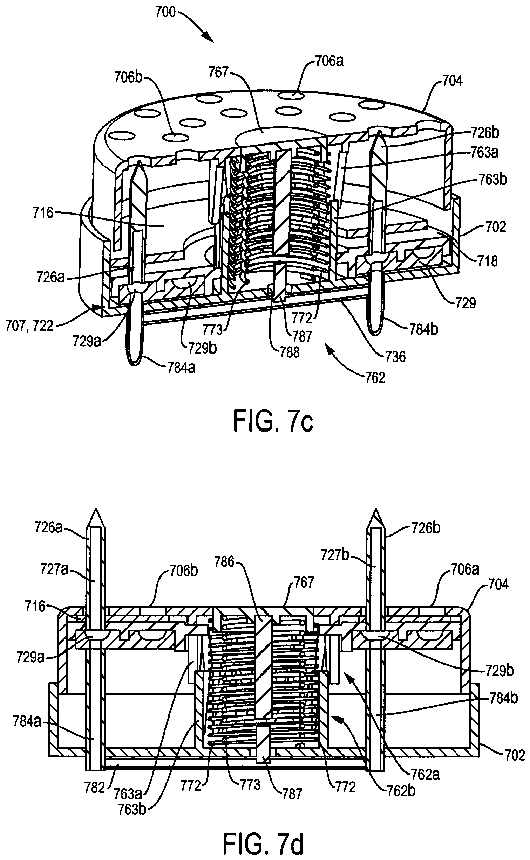

Referring to **FIGS. 7b** and **7c****,** inside the access port **700,** the needles **726a, 726b** may be supported by a needle support member **718,** which generally has a shape of an annular plate. A circular ratcheting mechanism **762** is disposed in the center of the access port **700,** which comprises an upper (top) annular cylindrical ratchet portion **762a** disposed above a lower (bottom) annular cylindrical ratchet portion **762b,** which engage and disengage from each other to change a circumferential (radial) position of the needles **726a, 726b.** The upper ratchet portion **762a** may have a cylindrical wall separate or integral to the cover **704,** particularly extending transverse to the top wall **704a** of the cover **704,** with a plurality of downward pointing teeth **763a.** Similarly, the lower ratchet portion **762b** may have a cylindrical wall separate or integral to the base **702,** particularly extending transverse to a bottom wall **702a** of the base **702,** with a plurality of upward pointing teeth **763b.**

The needle support member **718** contains circular channels **729a, 729b** in fluid communication with the lumens **727a, 727b** of needles **726a, 726b,** respectively, at the bottom surface of the needle support member **718,** and in fluid communication with the lumen of the catheters **784a, 784b,** respectively, that access the blood vessels of a patient. The circular channels may be defined by the bottom wall of the needle support member **718** in the form of two grooves in the bottom surface in which are fitted a circular ring **729** having also two circular grooves, hence defining channels **729a, 729b.**

Referring still to **FIG. 7b** and **7d****,** when an actuator **740** comprising a magnet **741,** such as a permanent magnet or an electromagnet, is applied by the medical personnel (e.g. physician, clinician) on the skin of the patient over the access port **700,** it activates the needle elevator mechanism **707** by attracting the permanent magnet **716** of the access port **700,** against the compression bias of outer coil spring **772** and raising the needle support member **718,** along with extending needles **726a, 726b** through respective openings **706a, 706b.** Removal of the permanent magnet, or reversal of the electromagnet **741,** will release the permanent magnet **716** and, with the decompression of outer coil spring **772** disposed inside the ratcheting mechanism, the needle support member **718** is returned to the bottom, with the needles **726a, 726b** retracted inside the access port **700.**

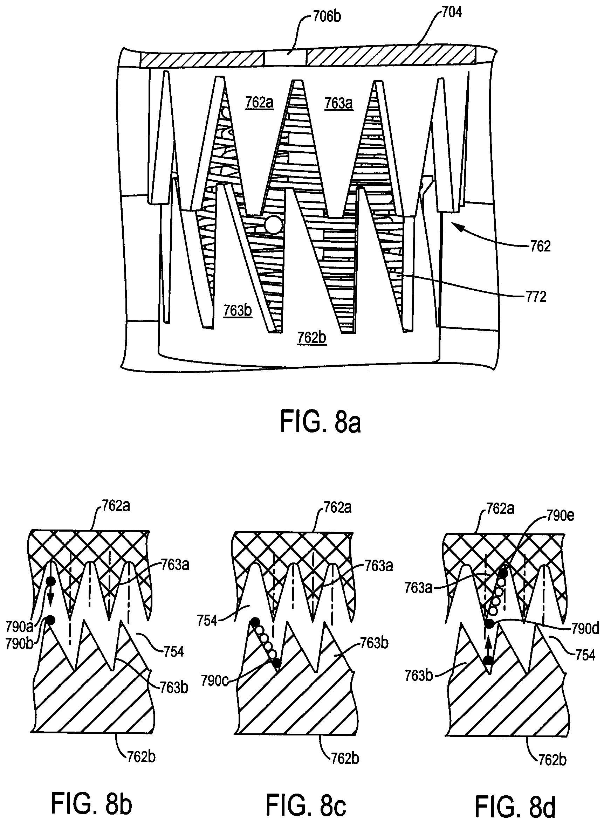

Medical personnel may then press the center button **767** to cause the teeth **763a** of the upper ratchet portion **762a** to engage with the teeth **763b** of the lower ratchet portion **762b,** forcing the lower ratchet portion **762b** to rotate one notch at a time for each use of the access port **700,** displacing with it the needle support member **718** circumferentially and thus positioning the needles **726a, 726b** at a new circumferential (radial) position as described in relation to **FIG. 8a** to **8d** below. The medical personnel have the option of repeatedly depressing button **767** in order to further advance rotation of the needles **726a, 726b** to other positions.

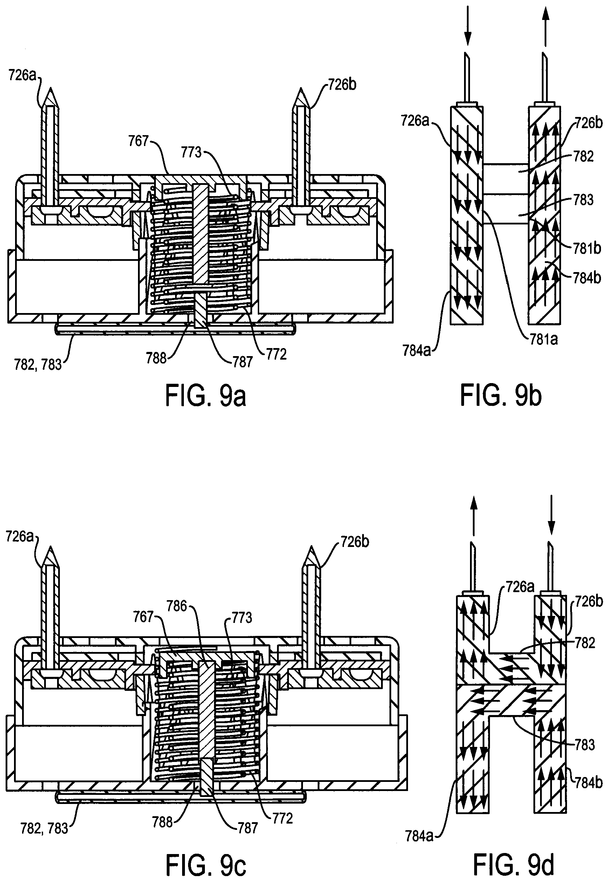

The details of the ratcheting mechanism are described in **FIG. 7d****.** The access port **700** may include a button **767** at the center of cover **704.** The button **767** is attached to a central push rod **786** and biased in the upward position by an inner coil spring **773.** When the button **767** is depressed, the central push rod **786** shifts down and pushes a shutter **787** that extends through an opening **788** in the base **702** and collapse the wall of the catheter bridges **782, 783** (only one shown) connecting both catheters **784a, 784b.** Push rod **786** is separate from shutter **787** to allow the button **767** to have a larger range of motion than required for shutter **787,** easing use by the medial personnel. The purpose and operation of this system is reviewed in greater details with regard to **FIG. 9a** to **9d** below.

**FIGS. 8a** to **8d** illustrate various stages of the operation of the ratcheting mechanism of access port **700.** As shown in **FIG. 8a****,** the upper ratchet portion **762a** and the lower ratchet portion **762b** of the ratcheting mechanism **762** are disposed around outer coil spring **772.** In FIGS. **8b-8d****,** dots **790a** to **790e** illustrate the rotation of the lower ratchet portion **762b** in relation to the upper ratchet portion **762a** upon a downward motion of the needles **726a, 726b** after an initial use of the access port **700.** As shown, during operation, the bottom teeth **763b** engage in an adjacent slot **754** between upper ratchet teeth **763a.** The ratcheting action forces the upper ratchet teeth **763a** to move the next position for engagement with the lower ratchet teeth **763b,** and the coil spring **772** creates a torsional force as it is compressed by button **767** that will drive the upper ratchet portion **762a** rotationally.

**FIGS. 9a** to **9d** illustrate the operation of the button **767** of access port **700** as shown in **FIG. 7d****.** As viewed in **FIGS. 9a** and **9b****,** the needles **726a, 726b** of the access port **700** are functioning in a blood flow mode. The button **767** is in an upper position flush with the top surface of the cover **704,** with central rod **786** being collinear with, but not engaging shutter **787.** Valve **781a** of needle **726a** is open and against the wall of the needle **726a,** and similarly, valve **781b** of needle **726b** is open and against the wall of the needle **726b.** In this configuration the blood circulates out of needle **726b,** and back into needle **726a** as when the access port **700** is used for hemodialysis of a patient with kidney failure.

As viewed in **FIG. 9c** and **9d****,** the needles **726a, 726b** of the access port **700** are functioning in a cleaning mode. The button **767** is in a depressed position. In this configuration the blood circulates in catheters **784a, 784b** through bottom bridge catheter **783** and a cleaning solution may be circulated in needles **726a, 726b** through upper bridge catheter **782.** Valve **781a** of needle **726a** is closed and blocking the flow between the needle **726a** and catheter **784a,** and similarly, valve **781b** of needle **726b** is closed and blocking the flow between the needle **726b** and catheter **784b.**

Valves **781a** and **781b** for connection to catheters **784a** and **784b** respectively may be of any sliding or rotational design incorporated into the body of the access port **700** following well known engineering principles to provide the fluid controls described in **FIG. 9b** and **FIG. 9d****.**

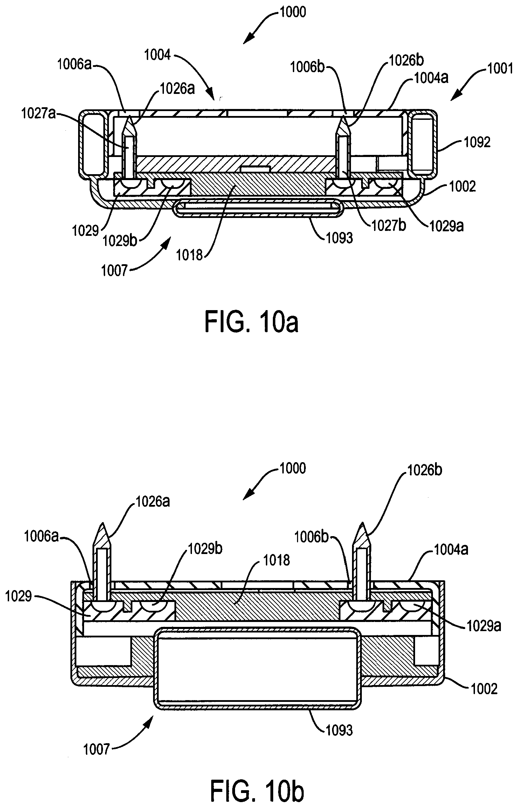

**FIGS. 10a** and **10b****,** illustrate another example of an access port **1000** with an alternate elevator mechanism **1007.** The access port **1000** may include a body **1001** comprising a cylindrical base **1002** supporting an overlying cylindrical cover **1004.** The cover **1004** may include on the top surface/wall **1004a** a series of predefined openings **1006a, 1006b** to allow the passage of needles **1026a, 1026b** respectively, through the top wall **1004a** of the cover **1004** to retract into and extend out of the body **1001** of the access port **1000.**

In order to prevent access of body fluids and/or ingrowth of tissue into the openings **1006a, 1006b** in the access port **1000,** the access port **1000** may also be contained in a housing **1003** (not shown, similar to housing **103**)**,** which may be made of a "self-healing" material, such as silicone. When extended, the needles **1026a, 1026b** may pierce through the self-healing material. Thereafter, when the needles **1026a, 1026b** are retracted, the openings **1006a, 1006b** created in the self-healing material may sufficiently close (i.e. self-close) so that the access port **1000** remains impervious to body fluids and ingrowth of body tissue. Alternatively, the cover **1004,** and more particularly the top wall **1004a** of the cover **1004,** may be made of a self-healing material, and the needles **1026a, 1026b** pierce through the top wall **1004a** of the cover **1004.**

Inside the access port **1000,** the needles **1026a, 1026b** may be supported by a needle support member **1018.** The needle support member **1018** contains circular channels **1029a, 1029b** in fluid communication with the lumens **1027a, 1027b** of needles **1026a, 1026b,** respectively, at the bottom surface of the needle support member **1018,** and to the lumen of the catheters that access the blood vessels of a patient (not shown). The circular channels **1029a, 1029b** may be defined by the bottom wall of the needle support member **1018** in the form of two grooves in the bottom surface in which are fitted a circular ring **1029** having also two circular grooves, hence defining channels **1029a, 1029b.**

In operation, the elevator mechanism **1007** of the vascular access port **1000** may be activated to raise the needles **1026a, 1026b** by medical personnel squeezing the sides of the vascular access port **1000,** on the side inflatable/deflatable balloons **1092** (as shown in **FIG. 10a**) shifting air or gas or a fluid contained therein to a bottom balloon **1093** (as shown in **FIG. 10b**). Inflation of balloon **1092** raises needle support member **1018,** forcing needles **1026a, 1026b** though openings **1006a, 1006b.** An opening **1094** at the center of the top wall **1004a** of the cover **1004,** may be used to place a ratcheting mechanism to move the circumferential (radial) position of the needles inside the access port **1000,** as well as a valve activating system to allow cleaning of the needle as previously described above.

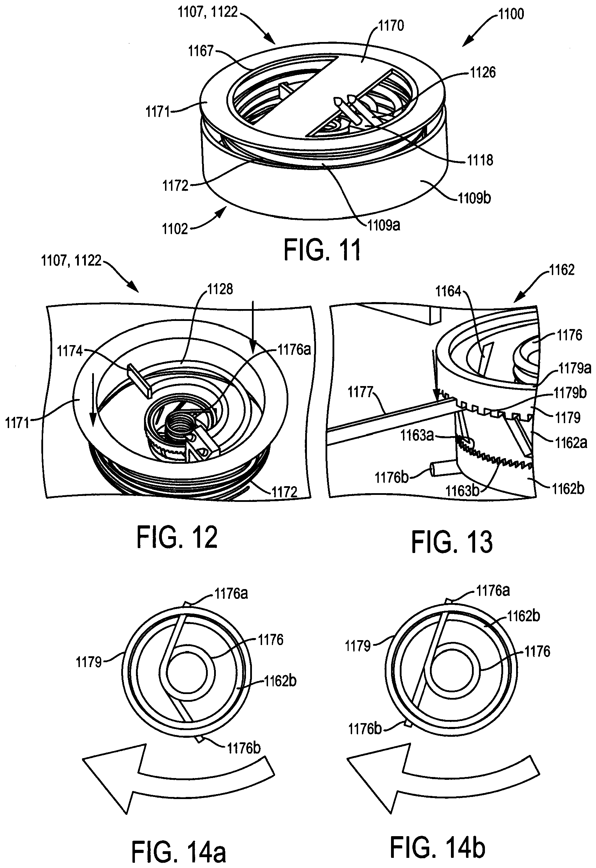

**FIGS. 11** to **13** illustrate another example of an access port **1100** with a needle elevator mechanism **1107** and needle shift mechanism **1122.** In this example, the cover has been removed to expose the needle elevator mechanism **1107** and needle shift mechanism **1122.** Cylindrical member **1109** may comprise an inner cylindrical member **1109a** and an outer cylindrical member **1109b** having a coil spring **1172** located there between. As shown, inner cylindrical member **1109a** has a flange **1171** disposed over coil spring **1172** such that coil spring **1172** biases downward movement of inner cylindrical member **1109a.**

Needle shift mechanism **1122** may more particularly be provided by a circular ratcheting mechanism **1162,** which may be activated by a button **1167** disposed in inner cylindrical member **1109a.** The button **1167** may include a cross-member/bridge **1170** that spans the diameter of the inner cylindrical member **1170** and is connected thereto by an annular ring. Inside the access port **1100,** the needles **1126** may be supported by a needle (arm) support member **1118** that is connected to a lower (bottom) annular cylindrical ratchet portion **1162b** of a ratcheting mechanism **1162.** Referring to **FIG. 12****,** the needle support member **1118** may contain channels connected to the needle shafts **1128** at one end and to the vascular catheters (not shown) at catheter inlets (not shown) at the other end. The needles **1126** may also include shafts **1128** supported by arm **1174.**

Referring to **FIG. 13****,** the ratcheting mechanism **1162** may also include an upper (top) ratchet portion **1162a** disposed above lower (bottom) annular cylindrical ratchet portion **1162b.** The upper (top) ratchet portion **1162a** may have a cylindrical wall with a series of elongated openings **1164** for receiving a bar **1177** which connects inner cylindrical member **1109a** to circular outer ratchet **1179** of the ratcheting mechanism **1162.** The ratcheting mechanism **1162** may include a torsion spring **1176,** with a top end **1176a** engaged in the outer ratchet **1179,** and the bottom end **1176b** engaged with the bottom ratchet portion **1162b.**

Referring to **FIGS. 12** and **13****,** when the flange **1171** is depressed by the medical personnel, it triggers the elevator mechanism **1107** of the ratcheting mechanism **1162** to shift (rotate) the position of the needles **1126** up through the needle support member **1118.** The ratcheting mechanism **1108** shifts (rotates) the needle support member **1118** clockwise forcing the needle shafts **1128** to slide through through-holes **1119** formed in the needle support member **1118.** Because the through-holes **1119** are oblique, the needles **1126** are elevated. When the button **1167** is depressed, it activates the needle-shift mechanism **1122** such that the bar **1177** slides in one of the elongated openings slits **1164,** rotating clockwise the upper (top) ratchet portion **1162a** as the bar **1177** shifts downward disengaging from teeth **1179b** the outer ratchet **1179.** The ratchet teeth **1163a** of the upper (top) ratchet portion **1162a** engage with the ratchet teeth **1163b** of the lower (bottom) ratchet portion **1162b** forcing both upper (top) ratchet portion **1162a** and lower (bottom) ratchet portion **1162b** to rotate together, displacing the needles **1126** out of the needle support member **1118.** Outer ratchet **1179** serves to prevent the torsion spring **1176** from releasing until sufficient energy is stored in the torsion spring **1176** to drive the needles **1126** upward.

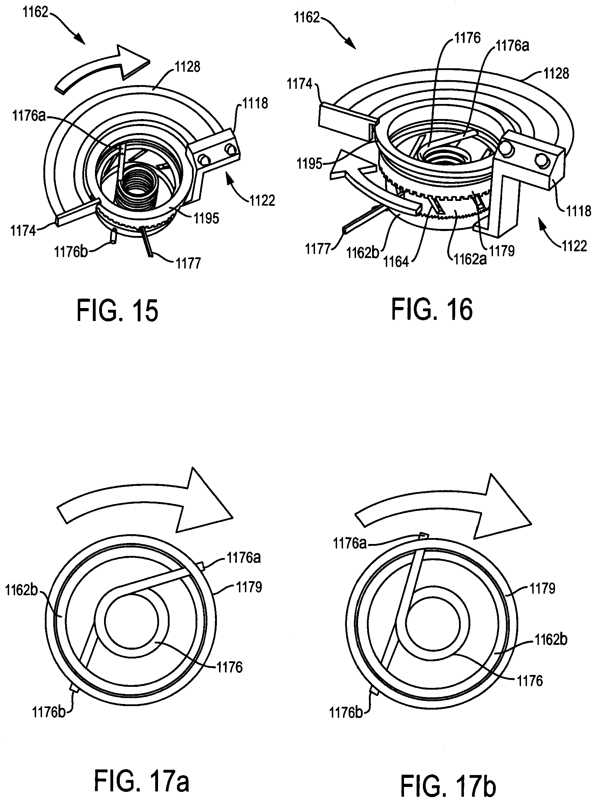

**FIGS. 14a** and **14b** illustrate top views of the configurations of a torsion spring used in the ratcheting mechanism as shown in **FIG. 12****.** When button **1167** is depressed, a clockwise rotational force of both the upper (top) ratchet portion **1162a** and lower (bottom) ratchet portion **1162b,** together, is applied to the bottom end **1176b** of the torsion spring **1176.** When button **1167** is depressed a sufficient number of times, the ratcheting mechanism **1162** will release the torsion spring **1176** to drive the needles **1126** upward. **FIGS. 14a** to **FIG. 18c** illustrate this process. When button **1167** is depressed lower (bottom) ratchet portion **1162b** is rotated clockwise relative to outer ratchet **1179,** which includes one end **1176b** of torsion spring **1176** causing energy to be stored in torsion spring **1176.** Referring to **FIG. 15****,** the needle shaft supporting arm **1174** is connected to an uppermost ring **1195** which provides access for cannula connections (not shown). Needles shafts **1128** schematically illustrate that a connection is to be provided between the supporting arm **1174** and needles **1126.** Needle shafts **1128** may be made of a suitable flexible or extendable material to allow the movement of needle to occur.

**FIG. 16** illustrates a partial perspective view of the ratcheting mechanism **1162** as shown in **FIG. 12****,** with the needle **1126** in the retracted position. Outer ratchet **1179** rotates clockwise relative to upper (top) ratchet portion **1162a** as button **1167** is depressed causing the ratcheting mechanism **1162** to be driven.

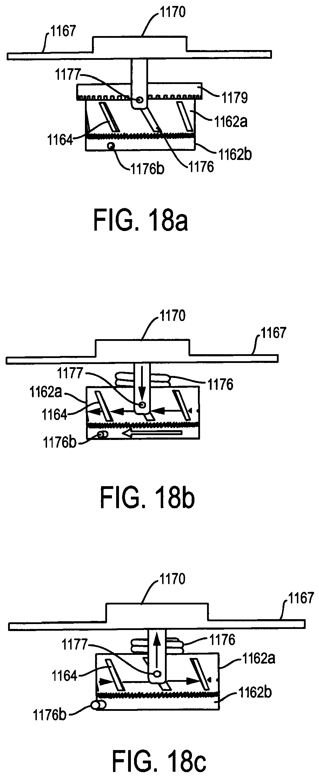

**FIGS. 17a** and **17b** illustrate top views of the configurations of a torsion spring **1176** used in the ratcheting mechanism **1162** as shown in **FIG. 12** when a clockwise rotation force by the outer ratchet **1179** is applied to the top end **1176a** of the torsion spring **1176.** When the torsion spring **1176** is sufficiently energized the ratcheting mechanism **1162** has rotated such that locking bar **1177** is released. The needles **1126** are driven forward by the movement of the torsion spring end **1176a** relative to the other end **1176b** of the torsion spring **1176,** causing the needles to move out of the port body **1101.** **FIGS. 18a** to **18c** illustrate side views of the configurations of the ratcheting mechanism **1162** as shown in **FIG. 12****.**

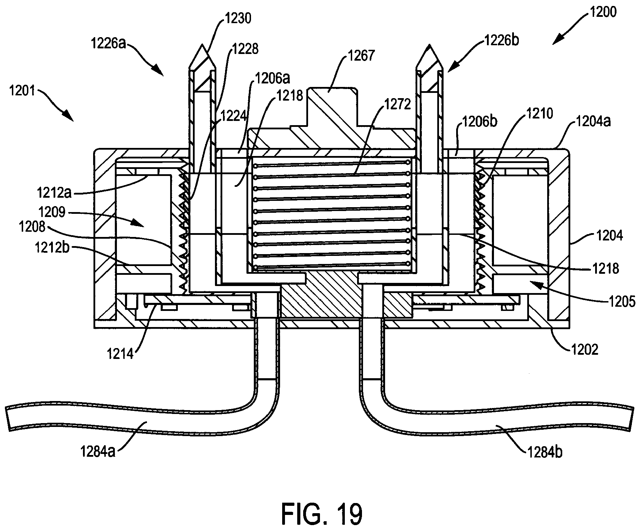

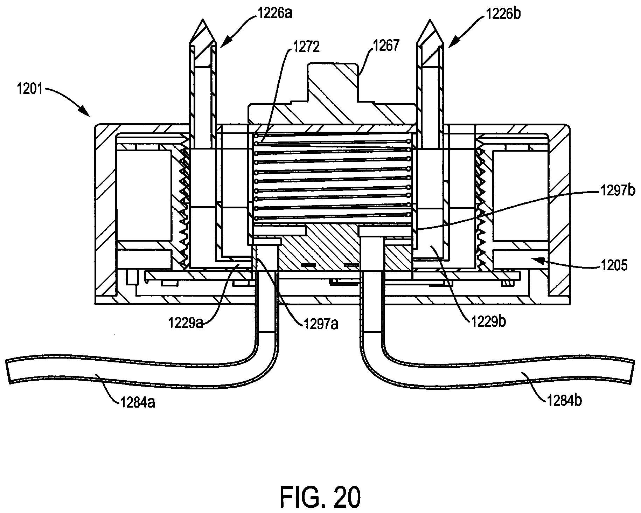

**FIGS. 19** and **20** illustrate the operation of an access port **1200** to open or close flow of fluid through the access port **1200.** **FIG. 19** illustrates the access port **1200** in an open configuration where fluid or blood can flow from the opening of the needles **1226** through the port and into the body catheters. The access port **1200** may include a body **1201** comprising a base **1202,** generally having a shape of a circular plate, supporting an overlying cover **1204,** which cooperate to form an inner cavity **1205.** The cover **1204** may have on the top surface/wall **1204a** a series of predefined openings **1206a, 1206b** to allow the passage of needles **1226a, 1226b** through the top wall **1204a** of the cover **1204** to retract into and extend out of the body **1201** of the access port **1200.**

In order to prevent access of body fluids and/or ingrowth of tissue into the openings **1206a, 1206b** in the access port **100,** the access port **1200** may be contained in a housing **1203** (not shown, similar to housing **103**), which may be made of a "self-healing" material, such as silicone. When extended, the needles **1226a, 1226b** may pierce through the self-healing material. Thereafter, when the needles **1226a, 1226b** are retracted, the openings **1206a, 1206b** created in the self-healing material may sufficiently close (i.e. self-close) so that the access port **1200** remains impervious to body fluids and ingrowth of body tissue. Alternatively, the cover **1204,** and more particularly the top wall **1204a** of the cover **1204,** may be made of a self-healing material, and the needles **1226a, 1226b** may pierce through the top wall **1204a** cover **1204.** The openings **1206a, 1206b** created in the self-healing material closes upon retraction of the needles **1226a, 1226b** so that the access port **1200** remains impervious to body fluids and ingrowth of body tissue.

As with previous embodiments, the cavity **1205** of the access port **1200** contains a needle elevator mechanism **1207,** which comprises a cylindrical (elevator) member **1209,** which rests and rotates on circular floor **1214.** As explained with previous embodiments, the floor **1214** of the needle elevator mechanism **1207** may also operate as part of a needle shift mechanism **1222.** The base **1202** supports floor **1214** and provides the structural foundation for the needle elevator mechanism **1207** and the needle shift mechanism **1222,** as well as forms part of the needle shift mechanism **1222.**

Cylindrical (elevator) member **1209** of the needle elevator mechanism **1207** comprises a cylinder **1208** which may include internal threads **1210,** as well as outer peripheral flanges **1212,** provided by upper flange **1212a** and lower flange **1212b.** The flanges **1212a, 1212b** may be used to hold at least one permanent magnet **1216** in a fixed position there between.

The needle elevator mechanism **1207** further comprises a needle support member **1218.** The access port needles **1226a, 1226b** are secured to (fastened) and supported within the interior of the cylinder **1208** by the needle support member **1218,** which generally has a shape of an annular plate. The needle support member **1218** may have external threads **1224** on the periphery that engages the internal threads **1210** of the cylinder **1208.** Each needle **1226a, 1226b** may include a shaft **1228** for connecting through a fluid path with internal catheters **1284a, 1284b** accessing the blood vessel, and removable a tip **1230** which allows access to the lumen **1227** of the needles **1226a, 1226b** for the passage of fluids or blood.

**FIG. 20** illustrates the access port **1200** in a close configuration where fluid or blood cannot flow through the access port **1200.** At the center of the vascular access port **1200,** a valve mechanism may be used by the medical personnel to open or close the flow of fluid through the access port **1200.** A coil spring **1272** may be compressed by depressing button **1267,** sliding valves **1297a, 1297b** to close channels **1229a, 1229b.**

The needles contemplated herein, may include any hollow cylinder or shaft. The needle may include, in some examples, standard bevels, short bevels, true short bevels, etc. Furthermore, the needles may exhibit an outer diameter in the range of 0.1 mm to 4.6 mm, including all values and increments therein. In addition, the needle may exhibit an inner diameter in the range of 0.08 mm to 4.0 mm, including all values and increments therein. Furthermore, the needles may exhibit a nominal wall thickness in the range of 0.002 mm to 0.4 mm including all values and increments therein. The needles may be formed of stainless steel, ceramic composites, or other materials. In addition, the needles or the needle tips may be replaceable in case of dulling.

Accordingly, a method of injecting a composition into a subject may be provided using the access port described herein. Once the port with at least one needle has been implanted in the patient and at least one internal catheter has been inserted into a vascular vein or body channel of a patient in need of the repeated systemic or local therapy, the access port may be accessed and therapy delivered according to the following steps. Medical personnel apply an actuator over the access port and activate the elevator mechanism in the direction that will raise the at least one needle out through the cover of the access port, piercing the skin at a first location. The actuator is put aside. The at least one needle is then connected to an syringe, or bag containing the composition through appropriate tubing or catheter. The composition is injected as a bolus or drip, or infused at the prescribed rate. Once the therapy has been delivered, the actuator is placed again over the access port and activated in a reversed direction that lowers the at least one needle under the skin and into the access port, under the cover. The actuator is maintained until the actuator engages the needle shift mechanism to displace the at least one needle from the position just used to a new position inside the access port such that when the access port is accessed again at the next therapy session, the needle will protrude at a new location.

A composition may include pharmaceuticals, nutrients, contrasting agents, blood or blood components, such as plasma, platelets, white blood cells, red blood cells, etc. Furthermore, a patient may include any vertebrate or invertebrate, including humans, other mammals, apes, domestic animals, cattle, etc. A vascular access port may be implanted into the patient and the catheter may be inserted into a vein. The needle may be extended from the port upon actuation and may puncture the skin. A composition may be introduced to the subject by either injecting the composition into the needle or otherwise introducing the needle into a container, such as through a vial stopper. Once administration of the composition is finished, the needle may be retracted or otherwise positioned back through the skin and into the port.

Alternatively, vascular access ports as described herein are suitable for use in hemodialysis of patient in need thereof, including patients in renal kidney failure and end stage renal disease. Once the vascular access port with at least two needles has been implanted in the patient and at least two internal catheters have been inserted into a vascular vein of a patient in need of the repeated hemodialysis, the port may be access and therapy performed according to the following steps. A medical personnel, apply an actuator over the vascular access port and activate the elevator mechanism in the direction that will raise the at least two needles out through the cover of the vascular access port, piercing the skin at a first location. The actuator is put aside. The at least two needles are then connected to a hemodialysis machine through appropriate tubing or catheters, one to receive the blood to be purified or filtered, the other to return the clean blood to the patient. Once the therapy has been delivered, the actuator is placed again over the vascular access port and activated in a reversed direction that lowers the at least two needles under the skin and into the vascular access port, under the cover. The actuator is maintained until the actuator engages the needle shift mechanism to displace the at least two needles from the position just used to a new position inside the vascular access port such that when the vascular access port is accessed again at the next therapy session, the needles will protrude at a new location.

The vascular access port described herein may be modified to define one of the possible circumferential (radial) positions of the needles to perform routine maintenance of the vascular access port. For example, the needles may be made of a conductive material, and when occupying the maintenance position, become connected to wiring that feeds a battery or a microprocessor in the vascular access port. The needles can then be connected to a power supply to recharge the vascular access port battery, to a computer for data transfer from a microprocessor in the vascular access port, or to control inputs for the operation of the vascular access port by a microprocessor.

The battery may be useful in a vascular access port that operates the needles extraction/retraction as well as rotation through a motor. Also, the battery may power a drug dispensing pump or other such mechanism that provides a release of a composition to the patient. Batteries may also be used to power implantable sensors or devices to transmit or receive information that provide diagnostic information to a clinician or still further another implantable device. Such information provided may include operational information on the vascular port, such as the position of needles in the port, the number of uses the port has experienced, the time between uses, etc. A vascular port may include a microprocessor so as to provide storage and processing of such information, programmable control of flow through the port or other such operations, means of preventing inadvertent operation of the port by requiring recognition of security passwords or for other means that my provide useful interaction with the port, external devices or with the clinician, however indirectly.

Alternatively, the needles may include removable and replaceable tips that allow electrical connection inside the needle body. Such tips may be reused after appropriate cleaning or preferably exchanged for sterilized replacements. Electrical connections may be made directly with the tips or via a mechanism exposed after tips are removed.

One or more maintenance positions may be used also to deliver chemicals to resupply a reservoir in the vascular access port that time-releases medicine to the patient, or that feeds a chemical battery, such as a fuel cell. The needles can then be connected to a separate channel that leads to the reservoir, or battery. In some embodiments, the chemical may be a gas for use in establishing pressure, such as to operate a pump that time-releases drug to the patient.

Alternatively, a maintenance position may be used to deliver a device to the vascular access port, such as replacement of a battery, vascular access port parts, RFID chips, microprocessors, encapsulated drugs, and the like.

To perform a maintenance operation, medical personnel, apply an actuator over the vascular access port and activate the port in the direction which will engage the needle shift mechanism to position the at least one needle at the maintenance location. Then, the actuator is then set to engage the elevator mechanism to raise the at least one needle out through the cover of the vascular access port, piercing the skin at the maintenance location. The actuator is put aside. The at least one needle is then used to performed the required maintenance as described above. Once the maintenance operation has been performed, the actuator is placed again over the access port and activated in a reversed direction that lowers the at least one needle under the skin and into the access port, under the cover. The actuator is maintained to operate the actuator to engage the needle shift mechanism to displace the at least one needle from the position just used to a new position inside the port such that when the vascular access port is accessed again at a therapy session, the needle will protrude at a location designated for performing the required therapy.

The foregoing description of several methods and embodiments has been presented for purposes of illustration. It is not intended to be exhaustive or to limit the claims to the precise steps and/or forms disclosed, and obviously many modifications and variations are possible in light of the above teaching. For example, other elevator mechanisms have been previously described in US Patent No. 8, 377,034 which may be used in the vascular access port described herein interchangeably. It is intended that the scope of the invention be defined by the claims appended hereto.

## Claims

1. A medical device comprising:
a subcutaneous implantable access port (100, 200, 300, 500, 600, 700, 1000, 1100, 1200) having an access port body (101, 201, 601, 701, 1001, 1201) and at least one needle (126, 226, 326, 426, 526a, 526b, 626, 726a, 726b, 1026a, 1026b, 1126, 1226, 1226a, 1226b) having a closed removable needle tip (130, 230, 330, 430, 630, 1230) and a needle shaft (128, 228, 328, 428, 628, 1128, 1228) defining a needle lumen (127, 227, 327, 427, 727a, 727b, 1027a, 1027b, 1227);
the at least one needle (126, 226, 326, 426, 526a, 526b, 626, 726a, 726b, 1026a, 1026b, 1126, 1226, 1226a, 1226b) housed within the access port body (101, 201, 601, 701, 1001, 1201), the at least one needle (126, 226, 326, 426, 526a, 526b, 626, 726a, 726b, 1026a, 1026b, 1126, 1226, 1226a, 1226b) extendable and retractable relative to the access port body (101, 201, 601, 701, 1001, 1201); and
a needle shift mechanism (122, 222, 322, 622, 722, 1122, 1222) operable such that the at least one needle (126, 226, 326, 426, 526a, 526b, 626, 726a, 726b, 1026a, 1026b, 1126, 1226, 1226a, 1226b) is extendable from and retractable into the access port body (101, 201, 601, 701, 1001, 1201) at a plurality of positions of the access port body (101, 201, 601, 701, 1001, 1201);
wherein the needle (126, 226, 326, 426, 526a, 526b, 626, 726a, 726b, 1026a, 1026b, 1126, 1226, 1226a, 1226b) includes the needle shaft (128, 228, 328, 428, 628, 1128, 1228) for connecting a fluid path with an internal catheter accessing a blood vessel and the closed, removable needle tip (130, 230, 330, 430, 630, 1230) which allows access to the needle lumen (127, 227, 327, 427, 727a, 727b, 1027a, 1027b, 1227) for the passage of fluids; and wherein the needle lumen (127, 227, 327, 427, 727a, 727b, 1027a, 1027b, 1227) is closed by the removable needle tip (130, 230, 330, 430, 630, 1230) when the removable needle tip (130, 230, 330, 430, 630, 1230) is disposed on the needle shaft (128, 228, 328, 428, 628, 1128, 1228).

2. The device of claim 1 wherein:
the needle shift mechanism (122, 222, 322, 622, 722, 1122, 1222) is operable such that the at least one needle (126, 226, 326, 426, 526a, 526b, 626, 726a, 726b, 1026a, 1026b, 1126, 1226, 1226a, 1226b) is rotatable along an arcuate path defined by a substantially constant radius about an axis of rotation (RA), preferably the arcuate path extends at least 90, 180, 270 or 360 degrees around the axis of rotation (RA) and the at least one needle (126, 226, 326, 426, 526a, 526b, 626, 726a, 726b, 1026a, 1026b, 1126, 1226, 1226a, 1226b) is extendable from and retractable into the access port body (101, 201, 601, 701, 1001, 1201) by moving axially along the axis of rotation (RA).

3. The device of claim 2 wherein:
the arcuate path is defined by a substantially constant radius from the axis of rotation (RA).

4. The device of claim 2 or claim 3 wherein:
the plurality of positions of the access port body (101, 201, 601, 701, 1001, 1201) at which the at least one needle (126, 226, 326, 426, 526a, 526b, 626, 726a, 726b, 1026a, 1026b, 1126, 1226, 1226a, 1226b) is extendable from and retractable into the access port body (101, 201, 601, 701, 1001, 1201) are arranged along the arcuate path, preferably the plurality of positions are substantially equally spaced along the arcuate path.

5. The device of claim 2 or claim 3 or claim 4 wherein:
the needle shift mechanism (122, 222, 322, 622, 722, 1122, 1222) is configured such that the at least one needle (126, 226, 326, 426, 526a, 526b, 626, 726a, 726b, 1026a, 1026b, 1126, 1226, 1226a, 1226b) is rotatable along the arcuate path about an axis of rotation (RA) in only one direction.

6. The device of any one of claims 1 to 5 wherein:
the needle shift mechanism (122, 222, 322, 622, 722, 1122, 1222) comprises a ratcheting mechanism (162, 262, 362, 662, 762, 1162).

7. The device of any one of claims 1 to 6 wherein:
the access port body (101, 201, 601, 701, 1001, 1201) comprises a cover (104, 204, 304, 604, 704, 1004, 1204) or a septum; and
the at least one needle (126, 226, 326, 426, 526a, 526b, 626, 726a, 726b, 1026a, 1026b, 1126, 1226, 1226a, 1226b) is extendable and retractable through the cover (104, 204, 304, 604, 704, 1004, 1204) or the septum.

8. The device of any one of claims 1 to 7 wherein:
the access port body (101, 201, 601, 701, 1001, 1201) comprises a plurality of needle openings (106, 206, 606, 706a, 706b, 1006a, 1006b, 1206a, 1206b); and
the at least one needle (126, 226, 326, 426, 526a, 526b, 626, 726a, 726b, 1026a, 1026b, 1126, 1226, 1226a, 1226b) is extendable and retractable through each of the plurality of needle openings (106, 206, 606, 706a, 706b, 1006a, 1006b, 1206a, 1206b).

9. The device of claim 8 wherein:
the plurality of needle openings (106, 206, 606, 706a, 706b, 1006a, 1006b, 1206a, 1206b) comprise a first needle opening (706a, 1006a, 1206a) and a second needle opening (706b, 1006b, 1206b); and
the needle shift mechanism (122, 222, 322, 622, 722, 1122, 1222) is operable to move the at least one needle (126, 226, 326, 426, 526a, 526b, 626, 726a, 726b, 1026a, 1026b, 1126, 1226, 1226a, 1226b) from a first position in which the at least one needle (126, 226, 326, 426, 526a, 526b, 626, 726a, 726b, 1026a, 1026b, 1126, 1226, 1226a, 1226b) is aligned with the first needle opening (706a, 1006a, 1206a) to extend and retract through the first needle opening (706a, 1006a, 1206a) to a second position in which the at least one needle (126, 226, 326, 426, 526a, 526b, 626, 726a, 726b, 1026a, 1026b, 1126, 1226, 1226a, 1226b) is aligned with the second needle opening (706b, 1006b, 1206b) to extend and retract through the second needle opening (706b, 1006b, 1206b).

10. The device of any one of claims 1 to 9 wherein:
the at least one needle (126, 226, 326, 426, 526a, 526b, 626, 726a, 726b, 1026a, 1026b, 1126, 1226, 1226a, 1226b) having the removable tip (130, 230, 330, 430, 630, 1230) and the needle shaft (128, 228, 328, 428, 628, 1128, 1228) defining the needle lumen (127, 227, 327, 427, 727a, 727b, 1027a, 1027b, 1227) further comprises a first needle (526a, 726a, 1026a, 1226a) having a removable first needle tip (130, 230, 330, 430, 630, 1230) and a first needle shaft (128, 228, 328, 428, 628, 1128, 1228) defining a first needle lumen (127, 227, 327, 427, 727a, 727b, 1027a, 1027b, 1227), and a second needle (526b, 726b, 1026b, 1226b) having a removable second needle tip (130, 230, 330, 430, 630, 1230) and a second needle shaft (128, 228, 328, 428, 628, 1128, 1228) defining a second needle lumen (127, 227, 327, 427, 727a, 727b, 1027a, 1027b, 1227); and
the needle shift mechanism (122, 222, 322, 622, 722, 1122, 1222) is further operable such that the first needle (526a, 726a, 1026a, 1226a) is extendable from and retractable into the access port body (101, 201, 601, 701, 1001, 1201) at a plurality of first needle positions of the access port body (101, 201, 601, 701, 1001, 1201), and the second needle (526b, 726b, 1026b, 1226b) is extendable from and retractable into the access port body (101, 201, 601, 701, 1001, 1201) at a plurality of second needle positions of the access port.

11. The device of claim 10 wherein:
the needle shift mechanism (122, 222, 322, 622, 722, 1122, 1222) is operable such that the first needle (526a, 726a, 1026a, 1226a) and the second needle (526b, 726b, 1026b, 1226b) are rotatable along an arcuate path about an axis of rotation (RA).

12. The device of any one of claims 1 to 11 further comprising:
a needle elevator mechanism (107, 207, 307, 607, 707, 1007, 1107, 1207), comprising e.g. a ratcheting mechanism, at least one magnet or a balloon arrangement, operable to extend the at least one needle (126, 226, 326, 426, 526a, 526b, 626, 726a, 726b, 1026a, 1026b, 1126, 1226, 1226a, 1226b) from the access port body (101, 201, 601, 701, 1001, 1201) and retract the at least one needle (126, 226, 326, 426, 526a, 526b, 626, 726a, 726b, 1026a, 1026b, 1126, 1226, 1226a, 1226b) into the access port body (101, 201, 601, 701, 1001, 1201), preferably by rotating the needle elevator mechanism (107, 207, 307, 607, 707, 1007, 1107, 1207) about an axis of rotation (RA).

13. The device of claim 12 wherein:
the access port body (101, 201, 601, 701, 1001, 1201) comprises a cover (104, 204, 304, 604, 704, 1004, 1204) having at least one opening (106, 206, 606, 706a, 706b, 1006a, 1006b, 1206a, 1206b); and
the needle elevator mechanism (107, 207, 307, 607, 707, 1007, 1107, 1207) is operable to extend the at least one needle (126, 226, 326, 426, 526a, 526b, 626, 726a, 726b, 1026a, 1026b, 1126, 1226, 1226a, 1226b) from the access port body (101, 201, 601, 701, 1001, 1201) through the at least one opening (106, 206, 606, 706a, 706b, 1006a, 1006b, 1206a, 1206b) in the cover (104, 204, 304, 604, 704, 1004, 1204) and retract the at least one needle (126, 226, 326, 426, 526a, 526b, 626, 726a, 726b, 1026a, 1026b, 1126, 1226, 1226a, 1226b) into the access port body (101, 201, 601, 701, 1001, 1201) through the at least one opening (106, 206, 606, 706a, 706b, 1006a, 1006b, 1206a, 1206b) in the cover (104, 204, 304, 604, 704, 1004, 1204).

14. The device of any one of claims 1 to 13 wherein:
the needle shift mechanism (122, 222, 322, 622, 722, 1122, 1222) is rotatable around an axis (RA); and the at least one needle (126, 226, 326, 426, 526a, 526b, 626, 726a, 726b, 1026a, 1026b, 1126, 1226, 1226a, 1226b) is extendable and retractable axially along the axis (RA).

15. The device of claim 12 or claim 13 or claim 14 wherein:
the needle elevator mechanism (107, 207, 307, 607, 707, 1007, 1107, 1207) comprises an elevator member and a needle support member (118, 218, 318, 418, 618, 718, 1018, 1118, 1218) having the at least one needle (126, 226, 326, 426, 526a, 526b, 626, 726a, 726b, 1026a, 1026b, 1126, 1226, 1226a, 1226b) supported thereon; and
the elevator member is threadably engaged with the needle support member (118, 218, 318, 418, 618, 718, 1018, 1118, 1218) and rotatable relative to the needle support member (118, 218, 318, 418, 618, 718, 1018, 1118, 1218) to extend the at least one needle (126, 226, 326, 426, 526a, 526b, 626, 726a, 726b, 1026a, 1026b, 1126, 1226, 1226a, 1226b) from the access port body (101, 201, 601, 701, 1001, 1201) and retract the at least one needle (126, 226, 326, 426, 526a, 526b, 626, 726a, 726b, 1026a, 1026b, 1126, 1226, 1226a, 1226b) into the access port body (101, 201, 601, 701, 1001, 1201),
wherein the elevator member preferably comprises a cylindrical member (109, 209, 309a, 309b, 509, 509a, 509b, 1109, 1109a, 1109b, 1209) which is rotatable around the needle support member (118, 218, 318, 418, 618, 718, 1018, 1118, 1218) or a threaded rod which is rotatable in a threaded opening of the needle support member (118, 218, 318, 418, 618, 718, 1018, 1118, 1218).

16. The device of any one of claims 1 to 15 further comprising:
at least one valve (581a, 581b, 781a, 781b, 1297a, 1297b) located within the access port (100, 200, 300, 500, 600, 700, 1000, 1100, 1200) to control a flow of fluid through the access port (100, 200, 300, 500, 600, 700, 1000, 1100, 1200),
wherein the at least one needle (126, 226, 326, 426, 526a, 526b, 626, 726a, 726b, 1026a, 1026b, 1126, 1226, 1226a, 1226b) comprises at least two needles (526a, 526b, 726a, 726b, 1026a, 1026b, 1226a, 1226b); and
the at least one valve (581a, 581b, 781a, 781b, 1297a, 1297b) comprises a plurality of valves (581a, 581b, 781a, 781b, 1297a, 1297b); and
preferably further comprising:
a first channel (582, 782) bridging the at least two needles (526a, 526b, 726a, 726b, 1026a, 1026b, 1226a, 1226b), wherein at least one of the plurality of valves (581a, 581b, 781a, 781b, 1297a, 1297b) arranged to open and close a flow of fluid across the first channel (582, 782); and/or
at least two catheters (584a, 584b, 784a, 784b, 1284a, 1284b) and a second channel bridging (582, 783) the at least two catheters (584a, 584b, 784a, 784b, 1284a, 1284b), wherein
at least one of the plurality of valves (581a, 581b, 781a, 781b, 1297a, 1297b) arranged to open and close a flow of fluid across the second channel (582, 783).

## Patentansprüche

1. Medizinische Vorrichtung mit:
einem subkutanen, implantierbaren Zugangsport (100, 200, 300, 500, 600, 700, 1000, 1100, 1200) mit einem Zugangsportkörper (101, 201, 601, 701, 1001, 1201) und zumindest einer Nadel (126, 226, 326, 426, 526a, 526b, 626, 726a, 726b, 1026a, 1026b, 1126, 1226, 1226a, 1226b), die eine geschlossene entfernbare Nadelspitze (130, 230, 330, 430, 630, 1230) und einen Nadelschaft (128, 228, 328, 428, 628, 1128, 1228) aufweist, der ein Nadellumen (127, 227, 327, 427, 727a, 727b, 1027a, 1027b, 1227) definiert;
wobei die zumindest eine Nadel (126, 226, 326, 426, 526a, 526b, 626, 726a, 726b, 1026a, 1026b, 1126, 1226, 1226a, 1226b) in dem Zugangsportkörper (101, 201, 601, 701, 1001, 1201) gelagert ist, wobei die zumindest eine Nadel (126, 226, 326, 426, 526a, 526b, 626, 726a, 726b, 1026a, 1026b, 1126, 1226, 1226a, 1226b) gegenüber dem Zugangsportkörper (101, 201, 601, 701, 1001, 1201) ausziehbar und zurückziehbar ausgebildet ist; und
einem Nadelverschiebemechanismus (122, 222, 322, 622, 722, 1122, 1222), der derart bedienbar ist, dass die zumindest eine Nadel (126, 226, 326, 426, 526a, 526b, 626, 726a, 726b, 1026a, 1026b, 1126, 1226, 1226a, 1226b) an einer Vielzahl von Positionen des Zugangsportkörpers (101, 201, 601, 701, 1001, 1201) aus dem Zugangsportkörper (101, 201, 601, 701, 1001, 1201) herausziehbar und in diesen zurückziehbar ist;
wobei die Nadel (126, 226, 326, 426, 526a, 526b, 626, 726a, 726b, 1026a, 1026b, 1126, 1226, 1226a, 1226b) den Nadelschaft (128, 228, 328, 428, 628, 1128, 1228) aufweist, um einen Flüssigkeitsweg mit einem inneren Katheter, der Zugang zu einem Blutgefäß hat, und der geschlossenen entfernbaren Nadelspitze (130, 230, 330, 430, 630, 1230) zu verbinden, welche einen Zugang zu dem Nadellumen (127, 227, 327, 427, 727a, 727b, 1027a, 1027b, 1227) für den Durchfluss von Flüssigkeiten ermöglicht; und
wobei das Nadellumen (127, 227, 327, 427, 727a, 727b, 1027a, 1027b, 1227) von der entfernbaren Nadelspitze (130, 230, 330, 430, 630, 1230) verschlossen wird, wenn die entfernbare Nadelspitze (130, 230, 330, 430, 630, 1230) auf den Nadelschaft (128, 228, 328, 428, 628, 1128, 1228) aufgesetzt wird.

2. Vorrichtung nach Anspruch 1, wobei:
der Nadelverschiebemechanismus (122, 222, 322, 622, 722, 1122, 1222) derart bedienbar ist, dass die zumindest eine Nadel (126, 226, 326, 426, 526a, 526b, 626, 726a, 726b, 1026a, 1026b, 1126, 1226, 1226a, 1226b) entlang einer von einem im Wesentlichen konstanten Radius definierten bogenförmigen Bahn um eine Drehachse (RA) drehbar ist, wobei sich die bogenförmige Bahn zumindest 90, 180, 270 oder 360 Grad um die Drehachse (RA) herum erstreckt und die zumindest eine Nadel (126, 226, 326, 426, 526a, 526b, 626, 726a, 726b, 1026a, 1026b, 1126, 1226, 1226a, 1226b) durch eine axiale Bewegung entlang der Drehachse (RA) aus dem Zugangsportkörper (101, 201, 601, 701, 1001, 1201) herausziehbar und in diesen zurückziehbar ist.

3. Vorrichtung nach Anspruch 2, wobei:
die bogenförmige Bahn durch einen im Wesentlichen konstanten Radius von der Drehachse (RA) definiert ist.

4. Vorrichtung nach Anspruch 2 oder Anspruch 3, wobei:
die Vielzahl von Positionen des Zugangsportkörpers (101, 201, 601, 701, 1001, 1201), an denen die zumindest eine Nadel (126, 226, 326, 426, 526a, 526b, 626, 726a, 726b, 1026a, 1026b, 1126, 1226, 1226a, 1226b) aus dem Zugangsportkörper (101, 201, 601, 701, 1001, 1201) herausziehbar und in diesen zurückziehbar ist, entlang der bogenförmigen Bahn angeordnet sind, wobei die Vielzahl von Positionen vorzugsweise mit im Wesentlichen gleichem Abstand voneinander entlang der bogenförmigen Bahn angeordnet sind.

5. Vorrichtung nach Anspruch 2 oder Anspruch 3 oder Anspruch 4, wobei:
der Nadelverschiebemechanismus (122, 222, 322, 622, 722, 1122, 1222) derart ausgebildet ist, dass die zumindest eine Nadel (126, 226, 326, 426, 526a, 526b, 626, 726a, 726b, 1026a, 1026b, 1126, 1226, 1226a, 1226b) entlang der bogenförmigen Bahn um eine Drehachse (RA) in nur einer Richtung drehbar ist.

6. Vorrichtung nach einem der Ansprüche 1 bis 5, wobei:
der Nadelverschiebemechanismus (122, 222, 322, 622, 722, 1122, 1222) einen Ratschenmechanismus (162, 262, 362, 662, 762, 1162) aufweist.

7. Vorrichtung nach einem der Ansprüche 1 bis 6, wobei:
der Zugangsportkörper (101, 201, 601, 701, 1001, 1201) eine Abdeckung (104, 204, 304, 604, 704, 1004, 1204) oder ein Septum aufweist; und die zumindest eine Nadel (126, 226, 326, 426, 526a, 526b, 626, 726a, 726b, 1026a, 1026b, 1126, 1226, 1226a, 1226b) durch die Abdeckung (104, 204, 304, 604, 704, 1004, 1204) oder das Septum hindurch ausziehbar und zurückziehbar ausgebildet ist.

8. Vorrichtung nach einem der Ansprüche 1 bis 7, wobei:
der Zugangsportkörper (101, 201, 601, 701, 1001, 1201) eine Vielzahl von Nadelöffnungen (106, 206, 606, 706a, 706b, 1006a, 1006b, 1206a, 1206b) aufweist; und
die zumindest eine Nadel (126, 226, 326, 426, 526a, 526b, 626, 726a, 726b, 1026a, 1026b, 1126, 1226, 1226a, 1226b) durch jede der Vielzahl von Nadelöffnungen (106, 206, 606, 706a, 706b, 1006a, 1006b, 1206a, 1206b) hindurch ausziehbar und zurückziehbar ausgebildet ist.

9. Vorrichtung nach Anspruch 8, wobei:
die Vielzahl von Nadelöffnungen (106, 206, 606, 706a, 706b, 1006a, 1006b, 1206a, 1206b) eine erste Nadelöffnung (706a, 1006a, 1206a) und eine zweite Nadelöffnung (706b, 1006b, 1206b) aufweist; und
der Nadelverschiebemechanismus (122, 222, 322, 622, 722, 1122, 1222) derart bedienbar ist, dass er die zumindest eine Nadel (126, 226, 326, 426, 526a, 526b, 626, 726a, 726b, 1026a, 1026b, 1126, 1226, 1226a, 1226b) aus einer ersten Position, in welcher die zumindest eine Nadel (126, 226, 326, 426, 526a, 526b, 626, 726a, 726b, 1026a, 1026b, 1126, 1226, 1226a, 1226b) mit der ersten Nadelöffnung (706a, 1006a, 1206a) ausgerichtet ist, um durch die erste Nadelöffnung (706a, 1006a, 1206a) herausgezogen und wieder zurückgezogen zu werden, in eine zweite Position bewegt, in welcher die zumindest eine Nadel (126, 226, 326, 426, 526a, 526b, 626, 726a, 726b, 1026a, 1026b, 1126, 1226, 1226a, 1226b) mit der zweiten Nadelöffnung (706b, 1006b, 1206b) ausgerichtet ist, um durch die zweite Nadelöffnung (706b, 1006b, 1206b) herausgezogen und wieder zurückgezogen zu werden.

10. Vorrichtung nach einem der Ansprüche 1 bis 9, wobei:
die zumindest eine Nadel (126, 226, 326, 426, 526a, 526b, 626, 726a, 726b, 1026a, 1026b, 1126, 1226, 1226a, 1226b) mit der entfernbaren Spitze (130, 230, 330, 430, 630, 1230) und dem das Nadellumen (127, 227, 327, 427, 727a, 727b, 1027a, 1027b, 1227) definierenden Nadelschaft (128, 228, 328, 428, 628, 1128, 1228) ferner eine erste Nadel (526a, 726a, 1026a, 1226a) aufweist, die eine entfernbare erste Nadelspitze 130, 230, 330, 430, 630, 1230) und einen ein erstes Nadellumen (127, 227, 327, 427, 727a, 727b, 1027a, 1027b, 1227) definierenden ersten Nadelschaft (128, 228, 328, 428, 628, 1128, 1228) umfasst, und eine zweite Nadel (526b, 726b, 1026b, 1226b), die eine entfernbare zweite Nadelspitze 130, 230, 330, 430, 630, 1230) und einen ein zweites Nadellumen (127, 227, 327, 427, 727a, 727b, 1027a, 1027b, 1227) definierenden zweiten Nadelschaft (128, 228, 328, 428, 628, 1128, 1228) umfasst; und
der Nadelverschiebemechanismus (122, 222, 322, 622, 722, 1122, 1222) ferner derart bedienbar ist, dass die erste Nadel (526a, 726a, 1026a, 1226a) aus dem Zugangsportkörper (101, 201, 601, 701, 1001, 1201) an einer Vielzahl von ersten Nadelpositionen des Zugangsportkörpers (101, 201, 601, 701, 1001, 1201) ausziehbar und in diesen zurückziehbar ist, und die zweite Nadel (526b, 726b, 1026b, 1226b) an einer Vielzahl von zweiten Nadelpositionen des Zugangsports aus dem Zugangsportkörper (101, 201, 601, 701, 1001, 1201) ausziehbar und in diesen zurückziehbar ist.

11. Vorrichtung nach Anspruch 10, wobei:
der Nadelverschiebemechanismus (122, 222, 322, 622, 722, 1122, 1222) derart bedienbar ist, dass die erste Nadel (526a, 726a, 1026a, 1226a) und die zweite Nadel (526b, 726b, 1026b, 1226b) entlang einer bogenförmigen Bahn um eine Drehachse (RA) drehbar sind.

12. Vorrichtung nach einem der Ansprüche 1 bis 11, ferner aufweisend:
einen Nadelhebemechanismus (107, 207, 307, 607, 707, 1007, 1107, 1207) mit z.B. einem Ratschenmechanismus, zumindest einem Magneten oder einer Ballonanordnung, die derart bedienbar sind, dass sie die zumindest eine Nadel (126, 226, 326, 426, 526a, 526b, 626, 726a, 726b, 1026a, 1026b, 1126, 1226, 1226a, 1226b) aus dem Zugangsportkörper (101, 201, 601, 701, 1001, 1201) herausschieben und die zumindest eine Nadel (126, 226, 326, 426, 526a, 526b, 626, 726a, 726b, 1026a, 1026b, 1126, 1226, 1226a, 1226b) in den Zugangsportkörper (101, 201, 601, 701, 1001, 1201) wieder zurückziehen, vorzugsweise durch Drehen des Nadelhebemechanismus (107, 207, 307, 607, 707, 1007, 1107, 1207) um eine Drehachse (RA).

13. Vorrichtung nach Anspruch 12, wobei:
der Zugangsportkörper (101, 201, 601, 701, 1001, 1201) eine Abdeckung (104, 204, 304, 604, 704, 1004, 1204) mit zumindest einer Öffnung (106, 206, 606, 706a, 706b, 1006a, 1006b, 1206a, 1206b) aufweist; und
der Nadelhebemechanismus (107, 207, 307, 607, 707, 1007, 1107, 1207) derart bedienbar ist, dass er die zumindest eine Nadel (126, 226, 326, 426, 526a, 526b, 626, 726a, 726b, 1026a, 1026b, 1126, 1226, 1226a, 1226b) aus dem Zugangsportkörper (101, 201, 601, 701, 1001, 1201) durch die zumindest eine Öffnung (106, 206, 606, 706a, 706b, 1006a, 1006b, 1206a, 1206b) herausschiebt und die zumindest eine Nadel (126, 226, 326, 426, 526a, 526b, 626, 726a, 726b, 1026a, 1026b, 1126, 1226, 1226a, 1226b) durch die zumindest eine Öffnung (106, 206, 606, 706a, 706b, 1006a, 1006b, 1206a, 1206b) wieder in den Zugangsportkörper (101, 201, 601, 701, 1001, 1201) zurückzieht.

14. Vorrichtung nach einem der Ansprüche 1 bis 13, wobei:
der Nadelverschiebemechanismus (122, 222, 322, 622, 722, 1122, 1222) drehbar um eine Achse (RA) ausgebildet ist; und die zumindest eine Nadel (126, 226, 326, 426, 526a, 526b, 626, 726a, 726b, 1026a, 1026b, 1126, 1226, 1226a, 1226b) axial entlang der Achse (RA) ausziehbar und zurückziehbar ist.

15. Vorrichtung nach Anspruch 12 oder Anspruch 13 oder Anspruch 14, wobei:
der Nadelhebemechanismus (107, 207, 307, 607, 707, 1007, 1107, 1207) ein Hebeelement und ein Nadeltragelement (118, 218, 318, 418, 618, 718, 1018, 1118, 1218) aufweist, auf welchem die zumindest eine Nadel (126, 226, 326, 426, 526a, 526b, 626, 726a, 726b, 1026a, 1026b, 1126, 1226, 1226a, 1226b) gelagert ist; und
das Hebeelement in gewindemäßigem Eingriff mit dem Nadeltragelement (118, 218, 318, 418, 618, 718, 1018, 1118, 1218) und drehbar gegenüber dem Nadeltragelement (118, 218, 318, 418, 618, 718, 1018, 1118, 1218) ausgebildet ist, um die zumindest eine Nadel (126, 226, 326, 426, 526a, 526b, 626, 726a, 726b, 1026a, 1026b, 1126, 1226, 1226a, 1226b) aus dem Zugangsportkörper (101, 201, 601, 701, 1001, 1201) herauszuschieben und die zumindest eine Nadel (126, 226, 326, 426, 526a, 526b, 626, 726a, 726b, 1026a, 1026b, 1126, 1226, 1226a, 1226b) in den Zugangsportkörper (101, 201, 601, 701, 1001, 1201) wieder zurückzuziehen,
wobei das Hebeelement vorzugsweise ein zylindrisches Element (109, 209, 309a, 309b, 509, 509a, 509b, 1109, 1109a, 1109b, 1209) aufweist, das drehbar um das Nadeltragelement (118, 218, 318, 418, 618, 718, 1018, 1118, 1218) ist, oder eine Gewindestange, die drehbar in einer Gewindeöffnung des Nadeltragelementes (118, 218, 318, 418, 618, 718, 1018, 1118, 1218) ausgebildet ist.

16. Vorrichtung nach einem der Ansprüche 1 bis 15, ferner aufweisend:
zumindest ein Ventil (581a, 581b, 781a, 781b, 1297a, 1297b), das innerhalb des Zugangsports (100, 200, 300, 500, 600, 700, 1000, 1100, 1200) angeordnet ist, um einen Flüssigkeitsfluss durch den Zugangsport (100, 200, 300, 500, 600, 700, 1000, 1100, 1200) zu steuern,
wobei die zumindest eine Nadel (126, 226, 326, 426, 526a, 526b, 626, 726a, 726b, 1026a, 1026b, 1126, 1226, 1226a, 1226b) zumindest zwei Nadeln (526a, 526b, 726a, 726b, 1026a, 1026b, 1226a, 1226b) aufweist; und
das zumindest eine Ventil (581a, 581b, 781a, 781b, 1297a, 1297b) eine Vielzahl von Ventilen (581a, 581b, 781a, 781b, 1297a, 1297b) aufweist; und
vorzugsweise ferner aufweisend:
einen ersten Kanal (582, 782), der zwischen den zumindest zwei Nadeln (526a, 526b, 726a, 726b, 1026a, 1026b, 1226a, 1226b) eine Brücke bildet, wobei zumindest eines der Vielzahl von Ventilen (581a, 581b, 781a, 781b, 1297a, 1297b) dafür angeordnet ist, einen Flüssigkeitsfluss durch den ersten Kanal (582, 782) zu öffnen und zu schließen; und/oder
zumindest zwei Katheter (684a, 584b, 784a, 784b, 1284a, 1284b) und einen zweiten Kanal (582, 783), der zwischen den zumindest zwei Kathetern (684a, 584b, 784a, 784b, 1284a, 1284b) eine Brücke bildet, wobei
zumindest eines der Vielzahl von Ventilen (581a, 581b, 781a, 781b, 1297a, 1297b) dafür angeordnet ist, einen Flüssigkeitsfluss durch den zweiten Kanal (582, 783) zu öffnen und zu schließen.

## Revendications

1. Dispositif médical comprenant:
un port d'accès sous-cutané implantable (100, 200, 300, 500, 600, 700, 1000, 1100, 1200) ayant un corps de port d'accès (101, 201, 601, 701, 1001, 1201) et au moins une aiguille (126, 226, 326, 426, 526a, 526b, 626, 726a, 726b, 1026a, 1026b, 1126, 1226, 1226a, 1226b), qui comprend une pointe de l'aiguille fermée amovible (130, 230, 330, 430, 630, 1230) et une tige d'aiguille (128, 228, 328, 428, 628, 1128, 1228), qui définit une lumière d'aiguille (127, 227, 327, 427, 727a, 727b, 1027a, 1027b, 1227);
l'au moins une aiguille (126, 226, 326, 426, 526a, 526b, 626, 726a, 726b, 1026a, 1026b, 1126, 1226, 1226a, 1226b) logée à l'intérieur du corps de port d'accès (101, 201, 601, 701, 1001, 1201), l'au moins une aiguille (126, 226, 326, 426, 526a, 526b, 626, 726a, 726b, 1026a, 1026b, 1126, 1226, 1226a, 1226b) étant extensible et rétractable par rapport au corps de port d'accès (101, 201, 601, 701, 1001, 1201); et
un mécanisme de déplacement d'aiguille (122, 222, 322, 622, 722, 1122, 1222) fonctionnant de sorte que l'au moins une aiguille (126, 226, 326, 426, 526a, 526b, 626, 726a, 726b, 1026a, 1026b, 1126, 1226, 1226a, 1226b) est extensible à partir du et rétractable dans le corps de port d'accès (101, 201, 601, 701, 1001, 1201) à une pluralité de positions du corps de port d'accès (101, 201, 601, 701, 1001, 1201);
dans lequel l'aiguille (126, 226, 326, 426, 526a, 526b, 626, 726a, 726b, 1026a, 1026b, 1126, 1226, 1226a, 1226b) comprend la tige d'aiguille (128, 228, 328, 428, 628, 1128, 1228) pour relier une trajectoire de fluide à un cathéter intérieur, qui a un accès à un vaisseau sanguin, et la pointe de l'aiguille fermée amovible (130, 230, 330, 430, 630, 1230), qui permet un accès à la lumière d'aiguille (127, 227, 327, 427, 727a, 727b, 1027a, 1027b, 1227) pour le passage de fluides; et
dans lequel la lumière d'aiguille (127, 227, 327, 427, 727a, 727b, 1027a, 1027b, 1227) est fermée par la pointe de l'aiguille fermée amovible (130, 230, 330, 430, 630, 1230), si la pointe de l'aiguille fermée amovible (130, 230, 330, 430, 630, 1230) est disposée sur la tige d'aiguille (128, 228, 328, 428, 628, 1128, 1228).

2. Dispositif selon la revendication 1, dans lequel:
le mécanisme de déplacement d'aiguille (122, 222, 322, 622, 722, 1122, 1222) fonctionne de sorte que l'au moins une aiguille (126, 226, 326, 426, 526a, 526b, 626, 726a, 726b, 1026a, 1026b, 1126, 1226, 1226a, 1226b) est rotative le long d'une trajectoire arquée définie par un rayon essentiellement constant autour d'un axe de rotation (RA), et de préférence la trajectoire arquée s'étend à au moins 90, 180, 270 ou 360 degrés autour de l'axe de rotation (RA), et l'au moins une aiguille (126, 226, 326, 426, 526a, 526b, 626, 726a, 726b, 1026a, 1026b, 1126, 1226, 1226a, 1226b) est extensible à partir du et rétractable dans le corps de port d'accès (101, 201, 601, 701, 1001, 1201) en se déplaçant axialement le long de l'axe de rotation (RA).

3. Dispositif selon la revendication 2, dans lequel:
la trajectoire arquée est définie par un rayon essentiellement constant à partir de l'axe de rotation (RA).

4. Dispositif selon la revendication 2 ou la revendication 3, dans lequel:
la pluralité de positions du corps de port d'accès (101, 201, 601, 701, 1001, 1201), auxquelles l'au moins une aiguille (126, 226, 326, 426, 526a, 526b, 626, 726a, 726b, 1026a, 1026b, 1126, 1226, 1226a, 1226b) est extensible à partir du et rétractable dans le corps de port d'accès (101, 201, 601, 701, 1001, 1201) sont disposées le long de la trajectoire arquée, de préférence la pluralité de positions sont essentiellement également espacées le long de la trajectoire arquée.

5. Dispositif selon la revendication 2 ou la revendication 3 ou la revendication 4, dans lequel:
le mécanisme de déplacement d'aiguille (122, 222, 322, 622, 722, 1122, 1222) est configuré de sorte que l'au moins une aiguille (126, 226, 326, 426, 526a, 526b, 626, 726a, 726b, 1026a, 1026b, 1126, 1226, 1226a, 1226b) est rotative le long de la trajectoire arquée autour d'un axe de rotation (RA) dans seulement une direction.

6. Dispositif selon l'une quelconque des revendication 1 à 5, dans lequel:
le mécanisme de déplacement d'aiguille (122, 222, 322, 622, 722, 1122, 1222) comprend un mécanisme d'encliquetage(162, 262, 362, 662, 762, 1162).

7. Dispositif selon l'une quelconque des revendication 1 à 6, dans lequel:
le corps de port d'accès (101, 201, 601, 701, 1001, 1201) comprend une couverture (104, 204, 304, 604, 704, 1004, 1204) ou un septum; et
l'au moins une aiguille (126, 226, 326, 426, 526a, 526b, 626, 726a, 726b, 1026a, 1026b, 1126, 1226, 1226a, 1226b) est extensible et rétractable à travers la couverture (104, 204, 304, 604, 704, 1004, 1204) ou à travers le septum.

8. Dispositif selon l'une quelconque des revendication 1 à 7, dans lequel:
le corps de port d'accès (101, 201, 601, 701, 1001, 1201) comprend une pluralité d'ouvertures d'aiguille (106, 206, 606, 706a, 706b, 1006a, 1006b, 1206a, 1206b); et
l'au moins une aiguille (126, 226, 326, 426, 526a, 526b, 626, 726a, 726b, 1026a, 1026b, 1126, 1226, 1226a, 1226b) est extensible et rétractable à travers chacune de la pluralité d'ouvertures d'aiguille (106, 206, 606, 706a, 706b, 1006a, 1006b, 1206a, 1206b).

9. Dispositif selon la revendication 8, dans lequel:
la pluralité d'ouvertures d'aiguille (106, 206, 606, 706a, 706b, 1006a, 1006b, 1206a, 1206b) comprennent une première ouverture d'aiguille (706a, 1006a, 1206a) et une deuxième ouverture d'aiguille (706b, 1006b, 1206b); et
le mécanisme de déplacement d'aiguille (122, 222, 322, 622, 722, 1122, 1222) fonctionne de sorte qu'il déplace l'au moins une aiguille (126, 226, 326, 426, 526a, 526b, 626, 726a, 726b, 1026a, 1026b, 1126, 1226, 1226a, 1226b) d'une première position, dans laquelle l'au moins une aiguille (126, 226, 326, 426, 526a, 526b, 626, 726a, 726b, 1026a, 1026b, 1126, 1226, 1226a, 1226b) est alignée avec la première ouverture d'aiguille (706a, 1006a, 1206a) pour s'étendre et se rétracter à travers la première ouverture d'aiguille (706a, 1006a, 1206a) à une deuxième position, dans laquelle l'au moins une aiguille (126, 226, 326, 426, 526a, 526b, 626, 726a, 726b, 1026a, 1026b, 1126, 1226, 1226a, 1226b) est alignée avec la deuxième ouverture d'aiguille (706b, 1006b, 1206b) pour s'étendre et se rétracter à travers la deuxième ouverture d'aiguille (706b, 1006b, 1206b).

10. Dispositif selon l'une quelconque des revendication 1 à 9, dans lequel:
l'au moins une aiguille (126, 226, 326, 426, 526a, 526b, 626, 726a, 726b, 1026a, 1026b, 1126, 1226, 1226a, 1226b) ayant la pointe amovible (130, 230, 330, 430, 630, 1230) et la tige d'aiguille (128, 228, 328, 428, 628, 1128, 1228), qui définit la lumière d'aiguille (127, 227, 327, 427, 727a, 727b, 1027a, 1027b, 1227) comprend en outre une première aiguille (526a, 726a, 1026a, 1226a) ayant une première pointe d'aiguille amovible (130, 230, 330, 430, 630, 1230) et une première tige d'aiguille (128, 228, 328, 428, 628, 1128, 1228) définissant une première lumière d'aiguille (127, 227, 327, 427, 727a, 727b, 1027a, 1027b, 1227) et une deuxième aiguille (526b, 726b 1026b, 1226b) ayant une deuxième pointe d'aiguille amovible (130, 230, 330, 430, 630, 1230) et une deuxième tige d'aiguille (128, 228, 328, 428, 628, 1128, 1228) définissant une deuxième lumière d'aiguille (127, 227, 327, 427, 727a, 727b, 1027a, 1027b, 1227); et
le mécanisme de déplacement d'aiguille (122, 222, 322, 622, 722, 1122, 1222) fonctionne en outre de sorte que la première aiguille (526a, 726a, 1026a, 1226a) est extensible à partir du et rétractable dans le corps de port d'accès (101, 201, 601, 701, 1001, 1201) à une pluralité de positions de première aiguille du corps de port d'accès (101, 201, 601, 701, 1001, 1201) et la deuxième aiguille (526b, 726b 1026b, 1226b) est extensible à partir du et rétractable dans le corps de port d'accès (101, 201, 601, 701, 1001, 1201) à une pluralité de positions de deuxième aiguille du port d'accès.

11. Dispositif selon la revendication 10, dans lequel:
le mécanisme de déplacement d'aiguille (122, 222, 322, 622, 722, 1122, 1222) fonctionne de sorte que la première aiguille (526a, 726a, 1026a, 1226a) et la deuxième aiguille sont rotatives le long d'une trajectoire arquée autour d'un axe de rotation (RA).

12. Dispositif selon l'une quelconque des revendication 1 à 11, comprenant en outre:
un mécanisme élévateur (107, 207, 307, 607, 707, 1007, 1107, 1207) comprenant par exemple un mécanisme d'encliquetage, au moins un aimant ou un ensemble de ballon fonctionnant de sorte qu'il sort l'au moins une aiguille (126, 226, 326, 426, 526a, 526b, 626, 726a, 726b, 1026a, 1026b, 1126, 1226, 1226a, 1226b) à partir du corps de port d'accès (101, 201, 601, 701, 1001, 1201) et rétracte l'au moins une aiguille (126, 226, 326, 426, 526a, 526b, 626, 726a, 726b, 1026a, 1026b, 1126, 1226, 1226a, 1226b) dans le corps de port d'accès (101, 201, 601, 701, 1001, 1201), de préférence en tournant le mécanisme élévateur d'aiguille (107, 207, 307, 607, 707, 1007, 1107, 1207) autour d'un axe de rotation (RA).

13. Dispositif selon la revendication 12, dans lequel:
le corps de port d'accès (101, 201, 601, 701, 1001, 1201) comprend une couverture (104. 204, 304, 604, 704, 1004, 1204) ayant au moins une ouverture (106, 206, 606, 706a, 706b, 1006a, 1006b, 1206a, 1206b); et
le mécanisme élévateur d'aiguille (107, 207, 307, 607, 707, 1007, 1107, 1207) fonctionne de sorte qu'il sort l'au moins une aiguille (126, 226, 326, 426, 526a, 526b, 626, 726a, 726b, 1026a, 1026b, 1126, 1226, 1226a, 1226b) à partir du corps de port d'accès (101, 201, 601, 701, 1001, 1201) à travers l'au moins une ouverture (106, 206, 606, 706a, 706b, 1006a, 1006b, 1206a, 1206b) dans la couverture (104. 204, 304, 604, 704, 1004, 1204) et rétracte l'au moins une aiguille (126, 226, 326, 426, 526a, 526b, 626, 726a, 726b, 1026a, 1026b, 1126, 1226, 1226a, 1226b) dans le corps de port d'accès (101, 201, 601, 701, 1001, 1201) à travers l'au moins une ouverture (106, 206, 606, 706a, 706b, 1006a, 1006b, 1206a, 1206b) dans la couverture (104. 204, 304, 604, 704, 1004, 1204).

14. Dispositif selon l'une quelconque des revendication 1 à 13, dans lequel:
le mécanisme de déplacement d'aiguille (122, 222, 322, 622, 722, 1122, 1222) peut tourner autour d'un axe (RA) ; et l'au moins une aiguille (126, 226, 326, 426, 526a, 526b, 626, 726a, 726b, 1026a, 1026b, 1126, 1226, 1226a, 1226b) est extensible et rétractable axialement le long de l'axe (RA).

15. Dispositif selon la revendication 12 ou la revendication 13 ou la revendication 14, dans lequel:
le mécanisme élévateur d'aiguille (107, 207, 307, 607, 707, 1007, 1107, 1207) comprend un membre élévateur et un membre de support d'aiguille (118, 218, 318, 418, 618, 718, 1018, 1118, 1218), qui supporte l'au moins une aiguille (126, 226, 326, 426, 526a, 526b, 626, 726a, 726b, 1026a, 1026b, 1126, 1226, 1226a, 1226b) sur celui-ci; et
le membre élévateur est relié de manière vissée au membre de support d'aiguille (118, 218, 318, 418, 618, 718, 1018, 1118, 1218) pour sortir l'au moins une aiguille (126, 226, 326, 426, 526a, 526b, 626, 726a, 726b, 1026a, 1026b, 1126, 1226, 1226a, 1226b) à partir du corps de port d'accès (101, 201, 601, 701, 1001, 1201) et pour rétracter l'au moins une aiguille (126, 226, 326, 426, 526a, 526b, 626, 726a, 726b, 1026a, 1026b, 1126, 1226, 1226a, 1226b) dans le corps de port d'accès (101, 201, 601, 701, 1001, 1201),
dans lequel le membre élévateur comprend de préférence un membre cylindrique (109, 209, 309a, 309b, 509, 509a, 509b, 1109, 1109a, 1109b, 1209), qui peut tourner autour du membre de support d'aiguille (118, 218, 318, 418, 618, 718, 1018, 1118, 1218) ou une tige filetée, qui peut tourner dans une ouverture filetée du membre de support d'aiguille (118, 218, 318, 418, 618, 718, 1018, 1118, 1218).

16. Dispositif selon l'une quelconque des revendication 1 à 15, comprenant en outre:
au moins une soupape (581a, 581b, 781a, 781b, 1297a, 1297b) logée à l'intérieur du port d'accès (100, 200, 300, 500, 600, 700, 1000, 1100, 1200) pour commander un flux de liquide à travers le port d'accès (100, 200, 300, 500, 600, 700, 1000, 1100, 1200),
dans lequel l'au moins une aiguille (126, 226, 326, 426, 526a, 526b, 626, 726a, 726b, 1026a, 1026b, 1126, 1226, 1226a, 1226b) comprend au moins deux aiguilles (526a, 526b, 726a, 726b, 1026a, 1026b, 1226a, 1226b); et
l'au moins une soupape (581a, 581b, 781a, 781b, 1297a, 1297b) comprend une pluralité de soupapes (581a, 581b, 781a, 781b, 1297a, 1297b); et
de préférence comprenant en outre:
un premier canal (582, 782) faisant un pont entre les au moins deux aiguilles (526a, 526b, 726a, 726b, 1026a, 1026b, 1226a, 1226b), dans lequel au moins une de la pluralité de soupapes (581a, 581b, 781a, 781b, 1297a, 1297b) est disposée pour ouvrir et fermer un flux de liquide à travers le premier canal (582, 782); et/oz
au moins deux cathéters (584a, 584b, 784a, 784b, 1284a, 1284b) et un deuxième canal (582, 783), qui fait le pont entre les au moins deux cathéters (584a, 584b, 784a, 784b, 1284a, 1284b), dans lequel
au moins une de la pluralité de soupapes (581a, 581b, 781a, 781b, 1297a, 1297b) est disposée pour ouvrir et fermer un flux de liquide à travers le deuxième canal (582, 783).
